(19) Europäisches Patentamt
European Patent Office
Office européen des brevets

(11) **EP 3 390 424 B1**

(12) **EUROPEAN PATENT SPECIFICATION**

(45) Date of publication and mention
of the grant of the patent:
**01.09.2021 Bulletin 2021/35**

(51) Int Cl.:
*C07K 1/13* (2006.01)          *C12N 9/90* (2006.01)
*G01N 33/532* (2006.01)      *G01N 33/58* (2006.01)
*G01N 33/68* (2006.01)

(21) Application number: **16822389.9**

(22) Date of filing: **13.12.2016**

(86) International application number:
**PCT/EP2016/080847**

(87) International publication number:
**WO 2017/102759 (22.06.2017 Gazette 2017/25)**

(54) **FKBP DOMAIN WITH TRANSGLUTAMINASE RECOGNITION SITE**

FKBP-DOMÄNE MIT TRANSGLUTAMINASE-ERKENNUNGSSTELLE

DOMAINE FKBP AVEC UN SITE DE RECONNAISSANCE DE TRANSGLUTAMINASE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.12.2015 EP 15200111**

(43) Date of publication of application:
**24.10.2018 Bulletin 2018/43**

(73) Proprietors:
• **Roche Diagnostics GmbH**
**68305 Mannheim (DE)**
Designated Contracting States:
**DE**
• **F. Hoffmann-La Roche AG**
**4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**

(72) Inventors:
• **SCHRAEML, Michael**
**82377 Penzberg (DE)**
• **STEFFEN, Wojtek**
**82418 Murnau (DE)**

(74) Representative: **Kuttenkeuler, David**
**Boehmert & Boehmert
Anwaltspartnerschaft mbB
Pettenkoferstrasse 22
80336 München (DE)**

(56) References cited:
WO-A1-2007/077008     WO-A1-2014/071978
WO-A1-2016/100735     WO-A2-00/43492
WO-A2-03/000878        WO-A2-2009/074318
WO-A2-2012/059882     CN-B- 103 421 753

• **GEITNER ANNE-JULIANE ET AL: "Generation of
a Highly Active Folding Enzyme by Combining a
Parvulin-Type Prolyl Isomerase from SurA with
an Unrelated Chaperone Domain", JOURNAL OF
MOLECULAR BIOLOGY, vol. 425, no. 22, 2013,
pages 4089-4098, XP028751688, ISSN: 0022-2836,
DOI: 10.1016/J.JMB.2013.06.038**
• **LOW C ET AL: "Crystal Structure Determination
and Functional Characterization of the
Metallochaperone SlyD from Thermus
thermophilus", JOURNAL OF MOLECULAR
BIOLOGY, ACADEMIC PRESS, UNITED
KINGDOM, vol. 398, no. 3, 7 May 2010
(2010-05-07), pages 375-390, XP027011890, ISSN:
0022-2836, DOI: 10.1016/J.JMB.2010.03.014
[retrieved on 2010-03-15] cited in the application**
• **KOVERMANN M ET AL: "Molecular function of the
prolyl cis/trans isolerase and metallochaperone
SlyD", BIOLOGICAL CHEMISTRY, WALTER DE
GRUYTER GMBH & CO, BERLIN, DE, vol. 394, no.
8, 1 August 2013 (2013-08-01), pages 965-975,
XP009190300, ISSN: 1431-6730 cited in the
application**

- **SCHLAPSCHY MARTIN ET AL: "Periplasmic chaperones used to enhance functional secretion of proteins in E. coli", METHODS IN MOLECULAR BIOLOGY, HUMANA PRESS, INC, US, vol. 705, 1 January 2011 (2011-01-01), pages 211-224, XP009146613, ISSN: 1064-3745, DOI: 10.1007/978-1-61737-967-3_12**
- **KNAPPE ET AL: "Insertion of a Chaperone Domain Converts FKBP12 into a Powerful Catalyst of Protein Folding", JOURNAL OF MOLECULAR BIOLOGY, ACADEMIC PRESS, UNITED KINGDOM, vol. 368, no. 5, 26 April 2007 (2007-04-26), pages 1458-1468, XP022046689, ISSN: 0022-2836, DOI: 10.1016/J.JMB.2007.02.097 cited in the application**

SEQUENCE LISTING

<110> Roche Diagnostics GmbH F. Hoffmann-La Roche AG Roche Diagnostics Operations, Inc.

<120> FKBP domain with transglutaminase recognition site

<130> R30521WO

<150> EP15200111.1
<151> 2015-12-15

<160> 65

<170> PatentIn version 3.5

<210> 1
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 1

```
                              Xaa Tyr Arg Tyr Arg Gln Xaa
                              1               5
```

<210> 2
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 2

```
                              Xaa Arg Tyr Arg Gln Arg Xaa
                              1               5
```

<210> 3
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 3

```
                              Xaa Arg Tyr Ser Gln Arg Xaa
                              1               5
```

<210> 4
<211> 7
<212> PRT
<213> Artificial Sequence

<220>
<223> Motif for Q-tag

<220>

<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 4

```
                              Xaa Phe Arg Gln Arg Gln Xaa
                              1                5
```

<210> 5
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 5

```
                              Xaa Arg Gln Arg Gln Arg Xaa
                              1                5
```

<210> 6
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 6

EP 3 390 424 B1

Xaa Phe Arg Gln Arg Gly Xaa
1                       5

<210> 7
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 7

Xaa Gln Arg Gln Arg Gln Xaa

1                       5

<210> 8
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 8

Xaa Tyr Lys Tyr Arg Gln Xaa
1                       5

<210> 9
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 9

```
                              Xaa Gln Tyr Arg Gln Arg Xaa
                              1                   5
```

<210> 10
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 10

```
                              Xaa Tyr Arg Gln Thr Arg Xaa
                              1                   5
```

<210> 11
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7) .. (7)
<223> Xaa can be any naturally occurring amino acid

<400> 11

```
                          Xaa Leu Arg Tyr Arg Gln Xaa
                          1                   5
```

<210> 12
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 12

```
                          Xaa Tyr Arg Gln Ser Arg Xaa
                          1                   5
```

<210> 13
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 13

```
                          Xaa Tyr Gln Arg Gln Arg Xaa
                          1                   5
```

<210> 14
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 14

```
                              Xaa Arg Tyr Thr Gln Arg Xaa
                              1               5
```

<210> 15
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 15

```
                              Xaa Arg Phe Ser Gln Arg Xaa
                              1               5
```

<210> 16
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 16

```
                         Xaa Gln Arg Gln Thr Arg Xaa
                         1               5
```

<210> 17
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 17

```
                         Xaa Trp Gln Arg Gln Arg Xaa
                         1               5
```

<210> 18
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 18

```
                         Xaa Pro Arg Tyr Arg Gln Xaa
                         1               5
```

<210> 19
<211> 7
<212> PRT
<213> Artificial Sequence

<220>
<223> Motif for Q-tag

<220>

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 19

```
                              Xaa Ala Tyr Arg Gln Arg Xaa
                              1               5
```

<210> 20
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 20

```
                              Xaa Val Arg Tyr Arg Gln Xaa
                              1               5
```

<210> 21
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 21

```
Xaa Val Arg Gln Arg Gln Xaa
1               5
```

<210> 22
<211> 7
<212> PRT
<213> Artificial Sequence

<220>
<223> Motif for Q-tag

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<400> 22

```
Xaa Tyr Arg Gln Arg Ala Xaa
1               5
```

<210> 23
<211> 262
<212> PRT
<213> Kutzneria albida

<400> 23

```
Xaa Val Arg Gln Arg Gln Xaa
1               5
```

```
Met His Lys Trp Phe Leu Arg Ala Ala Val Val Ala Ala Val Gly Phe
1               5                   10                  15

Gly Leu Pro Thr Leu Ile Ala Thr Thr Ala Gln Ala Ala Ala Val Ala
            20                  25                  30

Ala Pro Thr Pro Arg Ala Pro Leu Ala Pro Pro Leu Ala Glu Asp Arg
            35                  40                  45

Ser Tyr Arg Thr Trp Arg Val Glu Asp Tyr Val Glu Ala Trp Glu Arg
        50                  55                  60

Tyr His Gly Arg Glu Met Thr Glu Asp Glu Arg Glu Asn Leu Ala Arg
65                  70                  75                  80

Gly Cys Ile Gly Val Thr Val Val Asn Leu Asn Arg Glu Asp Leu Ser
                85                  90                  95

Asn Pro Pro Leu Asn Leu Ser Phe Gly Ser Leu Arg Thr Ala Glu Ala
            100                 105                 110

Val Gln Ala Ala Leu Asn Lys Ile Val Asp Thr His Pro Ser Pro Ala
        115                 120                 125

Gln Tyr Glu Ala Ala Val Ala Lys Asp Pro Ile Leu Lys Arg Leu Lys
    130                 135                 140

Asn Val Val Lys Ala Leu Pro Ser Trp Ile Asp Ser Ala Lys Leu Lys
145                 150                 155                 160

Ala Ser Ile Phe Ser Lys Arg Phe Tyr Ser Trp Gln Asn Pro Asp Trp
                165                 170                 175

Ser Glu Glu Arg Ala His Thr Thr Tyr Arg Pro Asp Arg Glu Thr Asp
            180                 185                 190

Gln Val Asp Met Ser Thr Tyr Arg Tyr Arg Ala Arg Pro Gly Tyr Val
            195                 200                 205

Asn Phe Asp Tyr Gly Trp Phe Asp Gln Asp Thr Asn Thr Trp Trp His
    210                 215                 220
```

```
Ala Asn His Glu Glu Pro Arg Met Val Val Tyr Gln Ser Thr Leu Arg
225             230             235             240

His Tyr Ser Arg Pro Leu Gln Asp Phe Asp Glu Gln Val Phe Thr Val
            245             250             255

Ala Phe Ala Lys Lys Asp
            260
```

<210> 24
<211> 196
<212> PRT
<213> Escherichia coli

<400> 24

```
Met Lys Val Ala Lys Asp Leu Val Val Ser Leu Ala Tyr Gln Val Arg
1               5               10              15

Thr Glu Asp Gly Val Leu Val Asp Glu Ser Pro Val Ser Ala Pro Leu
            20              25              30

Asp Tyr Leu His Gly His Gly Ser Leu Ile Ser Gly Leu Glu Thr Ala
        35              40              45

Leu Glu Gly His Glu Val Gly Asp Lys Phe Asp Val Ala Val Gly Ala
    50              55              60

Asn Asp Ala Tyr Gly Gln Tyr Asp Glu Asn Leu Val Gln Arg Val Pro
65              70              75              80

Lys Asp Val Phe Met Gly Val Asp Glu Leu Gln Val Gly Met Arg Phe
                85              90              95

Leu Ala Glu Thr Asp Gln Gly Pro Val Pro Val Glu Ile Thr Ala Val
            100             105             110

Glu Asp Asp His Val Val Val Asp Gly Asn His Met Leu Ala Gly Gln
        115             120             125

Asn Leu Lys Phe Asn Val Glu Val Val Ala Ile Arg Glu Ala Thr Glu
    130             135             140

Glu Glu Leu Ala His Gly His Val His Gly Ala His Asp His His His
145             150             155             160

Asp His Asp His Asp Gly Cys Cys Gly Gly His Gly His Asp His Gly
                165             170             175
```

```
His Glu His Gly Gly Glu Gly Cys Cys Gly Gly Lys Gly Asn Gly Gly
            180                 185                 190

Cys Gly Cys His
            195
```

<210> 25
<211> 176
<212> PRT
<213> Cyclobacteriaceae bacterium

<400> 25

```
Met Arg Phe Gln Ser Gln Leu Val Phe Met Glu Ile Ser Glu Asn Thr
1               5                   10                  15

Val Val Gly Leu Thr Tyr Glu Leu Lys Val Thr Asn Asn Glu Glu Asp
            20                  25                  30

Ser Ile Pro Phe Ser Val Glu Val Arg Asp Glu Glu Asp Pro Phe Tyr
            35                  40                  45

Phe Val Phe Gly Asn Ser Gly Leu Pro Glu Lys Phe Glu Arg Leu Leu
            50                  55                  60

Glu Asn Lys Val Ala Gly Asn Thr Phe Asn Phe Thr Leu Ser Ile Glu
65                  70                  75                  80

Glu Ala Tyr Gly His Ala Asp Glu Glu Leu Ile Leu Thr Val Pro Lys
            85                  90                  95

Lys Gln Phe Thr Gly Glu Lys Gly Phe Glu Pro Glu Met Leu Glu Glu
            100                 105                 110

Gly Asn Phe Leu Pro Leu Ile Asp Glu Asp Gly Tyr Pro Met Gln Ala
            115                 120                 125

Lys Val Ile Lys Asp Leu Gly Glu Glu Leu Leu Leu Asp Phe Asn His
            130                 135                 140

Pro Leu Val Gly Met Asn Leu His Phe Asp Gly Glu Val Tyr Lys Val
145                 150                 155                 160

Arg Lys Ala Thr Lys Glu Glu Thr Glu Lys Gly Tyr Ile Glu Val Asn
            165                 170                 175
```

<210> 26
<211> 176
<212> PRT

<213> Bacteroidales bacterium

<400> 26

```
Met Lys Ile Gly Thr Asn Lys Val Val Ser Leu Ala Tyr Thr Leu Glu
1               5                   10                  15

Val Glu Gly Asp Val Met Glu Thr Val Thr Ser Glu Lys Pro Leu Glu
            20                  25                  30

Phe Ile Phe Gly Thr Gly Tyr Leu Leu Pro Lys Phe Glu Glu Asn Val
            35                  40                  45

Ser Asn Lys Val Val Gly Asp Ala Phe Asp Phe Thr Leu Thr Ala Ser
        50                  55                  60

Glu Gly Tyr Gly Glu Glu Asn Pro Asp Ala Ile Ile Glu Leu Pro Lys
65                  70                  75                  80

Asp Ile Phe Lys Val Asp Gly Lys Ile Glu Glu Gly Leu Leu Thr Val
                85                  90                  95

Gly Asn Ile Leu Pro Met Gln Asp Ser Asp Gly Asn Arg Leu Gln Gly
            100                 105                 110

Ser Ile Asp Glu Ile Lys Asp Asp Val Val Val Met Asn Phe Asn His
            115                 120                 125

Pro Leu Ala Gly Ala Asp Leu His Phe Lys Gly Ile Val Val Ala Val
        130                 135                 140

Arg Glu Ala Ser Glu Thr Glu Leu Val Asn Gly Leu Arg Gly Glu Leu
145                 150                 155                 160

Gly Thr Ser Cys Gly Asp Gly Gly Cys Ser Gly Cys Ser Gly Cys His
                165                 170                 175
```

<210> 27
<211> 165
<212> PRT
<213> Escherichia coli

<400> 27

```
Met Lys Val Ala Lys Asp Leu Val Val Ser Leu Ala Tyr Gln Val Arg
1               5                   10                  15

Thr Glu Asp Gly Val Leu Val Asp Glu Ser Pro Val Ser Ala Pro Leu
            20                  25                  30

Asp Tyr Leu His Gly His Gly Ser Leu Ile Ser Gly Leu Glu Thr Ala
            35                  40                  45

Leu Glu Gly His Glu Val Gly Asp Lys Phe Asp Val Ala Val Gly Ala
        50                  55                  60

Asn Asp Ala Tyr Gly Gln Tyr Asp Glu Asn Leu Val Gln Arg Val Pro
65                  70                  75                  80

Lys Asp Val Phe Met Gly Val Asp Glu Leu Gln Val Gly Met Arg Phe
                85                  90                  95

Leu Ala Glu Thr Asp Gln Gly Pro Val Pro Val Glu Ile Thr Ala Val
            100                 105                 110

Glu Asp Asp His Val Val Val Asp Gly Asn His Met Leu Ala Gly Gln
            115                 120                 125

Asn Leu Lys Phe Asn Val Glu Val Val Ala Ile Arg Glu Ala Thr Glu
    130                 135                 140

Glu Glu Leu Ala His Gly His Val His Gly Ala His Asp His His His
145                 150                 155                 160

Asp His Asp His Asp
                165
```

<210> 28
<211> 149
<212> PRT
<213> Escherichia coli

<400> 28

```
Met Ser Glu Ser Val Gln Ser Asn Ser Ala Val Leu Val His Phe Thr
1               5                   10                  15

Leu Lys Leu Asp Asp Gly Thr Thr Ala Glu Ser Thr Arg Asn Asn Gly
            20                  25                  30

Lys Pro Ala Leu Phe Arg Leu Gly Asp Ala Ser Leu Ser Glu Gly Leu
            35                  40                  45

Glu Gln His Leu Leu Gly Leu Lys Val Gly Asp Lys Thr Thr Phe Ser
        50                  55                  60

Leu Glu Pro Asp Ala Ala Phe Gly Val Pro Ser Pro Asp Leu Ile Gln
65                  70                  75                  80

Tyr Phe Ser Arg Arg Glu Phe Met Asp Ala Gly Glu Pro Glu Ile Gly

                85                  90                  95

Ala Ile Met Leu Phe Thr Ala Met Asp Gly Ser Glu Met Pro Gly Val
            100                 105                 110

Ile Arg Glu Ile Asn Gly Asp Ser Ile Thr Val Asp Phe Asn His Pro
            115                 120                 125

Leu Ala Gly Gln Thr Val His Phe Asp Ile Glu Val Leu Glu Ile Asp
    130                 135                 140

Pro Ala Leu Glu Ala

145
```

<210> 29
<211> 149
<212> PRT
<213> Thermus thermophilus

<400> 29

```
Met Lys Val Gly Gln Asp Lys Val Val Thr Ile Arg Tyr Thr Leu Gln
1               5                   10                  15

Val Glu Gly Glu Val Leu Asp Gln Gly Glu Leu Ser Tyr Leu His Gly
            20                  25                  30

His Arg Asn Leu Ile Pro Gly Leu Glu Glu Ala Leu Glu Gly Arg Glu
            35                  40                  45

Glu Gly Glu Ala Phe Gln Ala His Val Pro Ala Glu Lys Ala Tyr Gly
        50                  55                  60

Pro His Asp Pro Glu Gly Val Gln Val Val Pro Leu Ser Ala Phe Pro
65                  70                  75                  80

Glu Asp Ala Glu Val Val Pro Gly Ala Gln Phe Tyr Ala Gln Asp Met
                85                  90                  95

Glu Gly Asn Pro Met Pro Leu Thr Val Val Ala Val Glu Gly Glu Glu
            100                 105                 110

Val Thr Val Asp Phe Asn His Pro Leu Ala Gly Lys Asp Leu Asp Phe
            115                 120                 125

Gln Val Glu Val Val Lys Val Arg Glu Ala Thr Pro Glu Glu Leu Leu
    130                 135                 140

His Gly His Ala His
```

```
                            145
```

<210> 30
<211> 7
<212> PRT
<213> Artificial Sequence

<220>
<223> Motif for Q-tag

<400> 30

```
                Gly Tyr Arg Tyr Arg Gln Gly
                1               5
```

<210> 31
<211> 7
<212> PRT
<213> Artificial Sequence

<220>

<223> Motif for Q-tag

<400> 31

```
Gly Arg Tyr Arg Gln Arg Gly
1               5
```

<210> 32
<211> 7
<212> PRT
<213> Artificial Sequence

<220>
<223> Motif for Q-tag

<400> 32

```
Gly Arg Tyr Ser Gln Arg Gly
1               5
```

<210> 33
<211> 7
<212> PRT
<213> Artificial Sequence

<220>
<223> Motif for Q-tag

<400> 33

```
Gly Phe Arg Gln Arg Gln Gly
1               5
```

<210> 34
<211> 7
<212> PRT
<213> Artificial Sequence

<220>
<223> Motif for Q-tag

<400> 34

```
Gly Arg Gln Arg Gln Arg Gly
1               5
```

<210> 35
<211> 7
<212> PRT
<213> Artificial Sequence

<220>
<223> Motif for Q-tag

<400> 35

```
Gly Phe Arg Gln Arg Gly Gly
1               5
```

<210> 36
<211> 7
<212> PRT
<213> Artificial Sequence

<220>
<223> Motif for Q-tag

<400> 36

```
Gly Gln Arg Gln Arg Gln Gly
1               5
```

<210> 37
<211> 7
<212> PRT
<213> Artificial Sequence

<220>
<223> Motif for Q-tag

<400> 37

```
Gly Tyr Lys Tyr Arg Gln Gly
1               5
```

<210> 38
<211> 7
<212> PRT
<213> Artificial Sequence

<220>
<223> Motif for Q-tag

<400> 38

```
Gly Gln Tyr Arg Gln Arg Gly

    1               5
```

<210> 39
<211> 7
<212> PRT
<213> Artificial Sequence

<220>
<223> Motif for Q-tag

<400> 39

```
Gly Tyr Arg Gln Thr Arg Gly
1               5
```

<210> 40
<211> 7
<212> PRT

<213> Artificial Sequence

<220>
<223> Motif for Q-tag

<400> 40

```
Gly Leu Arg Tyr Arg Gln Gly
1               5
```

<210> 41
<211> 7
<212> PRT
<213> Artificial Sequence

<220>
<223> Motif for Q-tag

<400> 41

```
Gly Tyr Arg Gln Ser Arg Gly
1               5
```

<210> 42
<211> 7
<212> PRT
<213> Artificial Sequence

<220>
<223> Motif for Q-tag

<400> 42

```
Gly Tyr Gln Arg Gln Arg Gly
1               5
```

<210> 43
<211> 7
<212> PRT
<213> Artificial Sequence

<220>
<223> Motif for Q-tag

<400> 43

```
Gly Arg Tyr Thr Gln Arg Gly
1               5
```

<210> 44
<211> 7
<212> PRT
<213> Artificial Sequence

<220>
<223> Motif for Q-tag

<400> 44

```
                                Gly Arg Phe Ser Gln Arg Gly
                                1               5
```

<210> 45
<211> 7
<212> PRT
<213> Artificial Sequence

<220>
<223> Motif for Q-tag

<400> 45

```
                                Gly Gln Arg Gln Thr Arg Gly
                                1               5
```

<210> 46
<211> 7
<212> PRT
<213> Artificial Sequence

<220>
<223> Motif for Q-tag

<400> 46

```
                                Gly Trp Gln Arg Gln Arg Gly
                                1               5
```

<210> 47
<211> 7
<212> PRT
<213> Artificial Sequence

<220>
<223> Motif for Q-tag

<400> 47

```
                                Gly Pro Arg Tyr Arg Gln Gly


                                   1               5
```

<210> 48
<211> 7
<212> PRT
<213> Artificial Sequence

<220>
<223> Motif for Q-tag

<400> 48

```
                                Gly Ala Tyr Arg Gln Arg Gly
                                1               5
```

<210> 49
<211> 7
<212> PRT
<213> Artificial Sequence

<220>
<223> Motif for Q-tag

<400> 49

```
Gly Val Arg Tyr Arg Gln Gly
1               5
```

<210> 50
<211> 7
<212> PRT
<213> Artificial Sequence

<220>
<223> Motif for Q-tag

<400> 50

```
Gly Val Arg Gln Arg Gln Gly
1               5
```

<210> 51
<211> 7
<212> PRT
<213> Artificial Sequence

<220>
<223> Motif for Q-tag

<400> 51

```
Gly Tyr Arg Gln Arg Ala Gly
1               5
```

<210> 52
<211> 117
<212> PRT
<213> Artificial Sequence

<220>
<223> Q-tag example

<400> 52

```
Met Lys Val Gly Gln Asp Lys Val Val Thr Ile Arg Tyr Thr Leu Gln
1               5                   10                  15

Val Glu Gly Glu Val Leu Asp Gln Gly Glu Leu Ser Tyr Leu His Gly
                20                  25                  30

His Arg Asn Leu Ile Pro Gly Leu Glu Glu Ala Leu Glu Gly Arg Glu
                35                  40                  45

Glu Gly Glu Ala Phe Gln Ala His Val Pro Ala Glu Lys Ala Tyr Gly
        50                  55                  60

Ala Gly Ser Gly Gly Gly Gly Arg Tyr Arg Gln Arg Gly Gly Gly Gly
65                  70                  75                  80

Gly Ser Ser Gly Lys Asp Leu Asp Phe Gln Val Glu Val Val Lys Val
                85                  90                  95

Arg Glu Ala Thr Pro Glu Glu Leu Leu His Gly His Ala His His His
                100                 105                 110

His His His His His
                115
```

<210> 53
<211> 6
<212> PRT
<213> Artificial Sequence

<220>
<223> K-tag example sequence

<400> 53

```
Arg Tyr Glu Ser Lys Gly
1               5
```

<210> 54
<211> 149
<212> PRT
<213> Artificial Sequence

<220>
<223> SlyD amino acid sequence

<400> 54

```
Met Lys Val Gly Gln Asp Lys Val Val Thr Ile Arg Tyr Thr Leu Gln
```

36

```
          1                    5                      10                       15

          Val Glu Gly Glu Val Leu Asp Gln Gly Glu Leu Ser Tyr Leu His Gly
                      20                  25                  30

          His Arg Asn Leu Ile Pro Gly Leu Glu Glu Ala Leu Glu Gly Arg Glu
                      35                  40                  45

          Glu Gly Glu Ala Phe Gln Ala His Val Pro Ala Glu Lys Ala Tyr Gly
                      50                  55                  60

          Pro His Asp Pro Glu Gly Val Gln Val Val Pro Leu Ser Ala Phe Pro
          65                  70                  75                  80

          Glu Asp Ala Glu Val Val Pro Gly Ala Gln Phe Tyr Ala Gln Asp Met
                              85                  90                  95

          Glu Gly Asn Pro Met Pro Leu Thr Val Val Ala Val Glu Gly Glu Glu
                      100                 105                 110

          Val Thr Val Asp Phe Asn His Pro Leu Ala Gly Lys Asp Leu Asp Phe
                      115                 120                 125

          Gln Val Glu Val Val Lys Val Arg Glu Ala Thr Pro Glu Glu Leu Leu
          130                 135                 140

          His Gly His Ala His
          145
```

<210> 55
<211> 110
<212> PRT
<213> Artificial Sequence

<220>
<223> SlyD with a MTG Q-tag

<400> 55

```
Met Lys Val Gly Gln Asp Lys Val Val Thr Ile Arg Tyr Thr Leu Gln
1               5                   10                  15

Val Glu Gly Glu Val Leu Asp Gln Gly Glu Leu Ser Tyr Leu His Gly
            20                  25                  30

His Arg Asn Leu Ile Pro Gly Leu Glu Glu Ala Leu Glu Gly Arg Glu
            35                  40                  45

Glu Gly Glu Ala Phe Gln Ala His Val Pro Ala Glu Lys Ala Tyr Gly
        50                  55                  60

Ala Gly Ser Gly Gly Gly Gly Asp Tyr Ala Leu Gln Gly Gly Gly Gly
65                  70                  75                  80

Gly Ser Ser Gly Lys Asp Leu Asp Phe Gln Val Glu Val Val Lys Val
                85                  90                  95

Arg Glu Ala Thr Pro Glu Glu Leu Leu His Gly His Ala His
            100                 105                 110
```

<210> 56
<211> 110
<212> PRT
<213> Artificial Sequence

<220>
<223> SlyD with a KalbTG Q-tag

<400> 56

38

```
Met Lys Val Gly Gln Asp Lys Val Val Thr Ile Arg Tyr Thr Leu Gln
1               5                   10                  15

Val Glu Gly Glu Val Leu Asp Gln Gly Glu Leu Ser Tyr Leu His Gly
            20                  25                  30

His Arg Asn Leu Ile Pro Gly Leu Glu Glu Ala Leu Glu Gly Arg Glu
            35                  40                  45

Glu Gly Glu Ala Phe Gln Ala His Val Pro Ala Glu Lys Ala Tyr Gly
        50                  55                  60

Ala Gly Ser Gly Gly Gly Gly Tyr Arg Tyr Arg Gln Gly Gly Gly Gly
65                  70                  75                  80

Gly Ser Ser Gly Lys Asp Leu Asp Phe Gln Val Glu Val Val Lys Val
                85                  90                  95

Arg Glu Ala Thr Pro Glu Glu Leu Leu His Gly His Ala His
            100                 105                 110
```

<210> 57
<211> 149
<212> PRT
<213> Artificial Sequence

<220>
<223> SlpA amino acid sequence

<400> 57

```
Met Ser Glu Ser Val Gln Ser Asn Ser Ala Val Leu Val His Phe Thr
```

```
          1                    5                      10                      15

      Leu Lys Leu Asp Asp Gly Thr Thr Ala Glu Ser Thr Arg Asn Asn Gly
                  20                      25                  30

      Lys Pro Ala Leu Phe Arg Leu Gly Asp Ala Ser Leu Ser Glu Gly Leu
                  35                      40                  45

      Glu Gln His Leu Leu Gly Leu Lys Val Gly Asp Lys Thr Thr Phe Ser
                  50                      55                  60

      Leu Glu Pro Asp Ala Ala Phe Gly Val Pro Ser Pro Asp Leu Ile Gln
      65                      70                      75                  80

      Tyr Phe Ser Arg Arg Glu Phe Met Asp Ala Gly Glu Pro Glu Ile Gly
                      85                      90                  95

      Ala Ile Met Leu Phe Thr Ala Met Asp Gly Ser Glu Met Pro Gly Val
                  100                     105                 110

      Ile Arg Glu Ile Asn Gly Asp Ser Ile Thr Val Asp Phe Asn His Pro
                  115                     120                 125

      Leu Ala Gly Gln Thr Val His Phe Asp Ile Glu Val Leu Glu Ile Asp
          130                     135                 140

      Pro Ala Leu Glu Ala
          145
```

<210> 58
<211> 189
<212> PRT
<213> Artificial Sequence

<220>
<223> main HTLV antigen and viral envelope glycoprotein amino acid sequence 'gp21'

<400> 58

```
Ile Val Ser Ser Ala Cys Asn Asn Ser Leu Ile Leu Pro Pro Phe Ser
1               5                   10                  15

Leu Ser Pro Val Pro Thr Val Gly Ser Arg Ser Arg Arg Ala Val Pro
            20                  25                  30

Val Ala Val Trp Phe Val Ser Ala Leu Ala Met Gly Ala Gly Val Ala
            35                  40                  45

Gly Gly Ile Thr Gly Ser Met Ser Leu Ala Ser Gly Lys Ser Leu Leu

    50                  55                  60

His Glu Val Asp Lys Asp Ile Ser Gln Leu Thr Gln Ala Ile Val Lys
65                  70                  75                  80

Asn His Lys Asn Leu Leu Lys Ile Ala Gln Tyr Ala Ala Gln Asn Arg
                85                  90                  95

Arg Gly Leu Asp Leu Leu Phe Trp Glu Gln Gly Gly Leu Cys Lys Ala
            100                 105                 110

Leu Gln Glu Gln Cys Cys Phe Leu Asn Ile Thr Asn Ser His Val Ser
            115                 120                 125

Ile Leu Gln Glu Arg Pro Pro Leu Glu Asn Arg Val Leu Thr Gly Trp
    130                 135                 140

Gly Leu Asn Trp Asp Leu Gly Leu Ser Gln Trp Ala Arg Glu Ala Leu
145                 150                 155                 160

Gln Thr Gly Ile Thr Leu Val Ala Leu Leu Leu Leu Val Ile Leu Ala
            165                 170                 175

Gly Pro Cys Ile Arg Cys Pro Cys Arg Thr Met His Pro
            180                 185
```

<210> 59
<211> 109
<212> PRT
<213> Artificial Sequence

<220>
<223> amino acid sequence of modified the 'gp21' ectodomain polypeptide sequence, engineered for better solubility, stability and reactivity in the immunoassay

<400> 59

```
Met Ser Leu Ala Ser Gly Lys Ser Leu Leu His Glu Val Asp Lys Asp
1               5                   10                  15

Ile Ser Gln Leu Thr Gln Ala Ile Val Lys Asn His Lys Asn Leu Leu
            20                  25                  30

Lys Ile Ala Gln Tyr Ala Ala Gln Asn Arg Arg Gly Leu Asp Leu Leu
            35                  40                  45

Phe Trp Glu Gln Gly Gly Leu Ala Lys Ala Leu Gln Glu Gln Ala Ala
    50                  55                  60

Phe Leu Asn Ile Thr Asn Ser His Val Ser Ile Leu Gln Glu Arg Pro
65                  70                  75                  80

Pro Leu Glu Asn Arg Val Leu Thr Gly Trp Gly Leu Asn Trp Asp Leu
                85                  90                  95

Gly Leu Ser Gln Trp Ala Arg Glu Ala Leu Gln Thr Gly
                100                 105
```

<210> 60
<211> 255
<212> PRT
<213> Artificial Sequence

<220>
<223> recombinant fusion protein 'TtSlyQD-Xa-gp21-8H'

<400> 60

Met Lys Val Gly Gln Asp Lys Val Val Thr Ile Arg Tyr Thr Leu Gln
1                   5                    10                    15

Val Glu Gly Glu Val Leu Asp Gln Gly Glu Leu Ser Tyr Leu His Gly
                20                    25                    30

His Arg Asn Leu Ile Pro Gly Leu Glu Glu Ala Leu Glu Gly Arg Glu
                35                    40                    45

Glu Gly Glu Ala Phe Gln Ala His Val Pro Ala Glu Lys Ala Tyr Gly
        50                    55                    60

Ala Gly Ser Gly Gly Gly Gly Asp Tyr Ala Leu Gln Gly Gly Gly Gly
65                    70                    75                    80

Gly Ser Ser Gly Lys Asp Leu Asp Phe Gln Val Glu Val Val Lys Val
                85                    90                    95

Arg Glu Ala Thr Pro Glu Glu Leu Leu His Gly His Ala His Gly Gly
                100                   105                   110

Gly Ser Gly Gly Gly Ser Gly Gly Gly Ser Gly Gly Gly Ser Gly Gly
        115                   120                   125

Gly Ser Gly Gly Gly Ile Glu Gly Arg Met Ser Leu Ala Ser Gly Lys
        130                   135                   140

Ser Leu Leu His Glu Val Asp Lys Asp Ile Ser Gln Leu Thr Gln Ala
145                   150                   155                   160

Ile Val Lys Asn His Lys Asn Leu Leu Lys Ile Ala Gln Tyr Ala Ala

                              165                    170                         175

        Gln Asn Arg Arg Gly Leu Asp Leu Leu Phe Trp Glu Gln Gly Gly Leu
                    180                     185              190

        Ala Lys Ala Leu Gln Glu Gln Ala Ala Phe Leu Asn Ile Thr Asn Ser
                195                     200              205

        His Val Ser Ile Leu Gln Glu Arg Pro Pro Leu Glu Asn Arg Val Leu
                210                     215              220

        Thr Gly Trp Gly Leu Asn Trp Asp Leu Gly Leu Ser Gln Trp Ala Arg
        225                     230              235                  240

        Glu Ala Leu Gln Thr Gly Gly His His His His His His His His
                    245                     250              255

<210> 61
<211> 294
<212> PRT
<213> Artificial Sequence

<220>
<223> recombinant fusion protein 'TtSlyD-Xa-gp21-8H'

<400> 61

```
Met Lys Val Gly Gln Asp Lys Val Val Thr Ile Arg Tyr Thr Leu Gln
1               5                   10                  15

Val Glu Gly Glu Val Leu Asp Gln Gly Glu Leu Ser Tyr Leu His Gly
            20                  25                  30

His Arg Asn Leu Ile Pro Gly Leu Glu Glu Ala Leu Glu Gly Arg Glu
        35                  40                  45

Glu Gly Glu Ala Phe Gln Ala His Val Pro Ala Glu Lys Ala Tyr Gly
        50                  55                  60

Pro His Asp Pro Glu Gly Val Gln Val Val Pro Leu Ser Ala Phe Pro
65                  70                  75                  80

Glu Asp Ala Glu Val Val Pro Gly Ala Gln Phe Tyr Ala Gln Asp Met
                85                  90                  95

Glu Gly Asn Pro Met Pro Leu Thr Val Val Ala Val Glu Gly Glu Glu
            100                 105                 110

Val Thr Val Asp Phe Asn His Pro Leu Ala Gly Lys Asp Leu Asp Phe
            115                 120                 125
```

```
Gln Val Glu Val Val Lys Val Arg Glu Ala Thr Pro Glu Glu Leu Leu
    130             135             140

His Gly His Ala His Gly Gly Gly Ser Gly Gly Gly Ser Gly Gly Gly
145             150             155             160

Ser Gly Gly Gly Ser Gly Gly Gly Ser Gly Gly Ile Glu Gly Arg
                165             170             175

Met Ser Leu Ala Ser Gly Lys Ser Leu Leu His Glu Val Asp Lys Asp
            180             185             190

Ile Ser Gln Leu Thr Gln Ala Ile Val Lys Asn His Lys Asn Leu Leu
            195             200             205

Lys Ile Ala Gln Tyr Ala Ala Gln Asn Arg Arg Gly Leu Asp Leu Leu
    210             215             220

Phe Trp Glu Gln Gly Gly Leu Ala Lys Ala Leu Gln Glu Gln Ala Ala
225             230             235             240

Phe Leu Asn Ile Thr Asn Ser His Val Ser Ile Leu Gln Glu Arg Pro
            245             250             255

Pro Leu Glu Asn Arg Val Leu Thr Gly Trp Gly Leu Asn Trp Asp Leu
    260             265             270

Gly Leu Ser Gln Trp Ala Arg Glu Ala Leu Gln Thr Gly Gly His His
    275             280             285

His His His His His His
        290
```

<210> 62
<211> 427
<212> PRT
<213> Artificial Sequence

<220>
<223> recombinant fusion protein 'TtSlyKQD-SlpA-Xa-gp21-8H'

<400> 62

```
Met Lys Val Gly Gln Asp Lys Val Val Thr Ile Arg Tyr Thr Leu Gln
1               5               10              15

Val Glu Gly Glu Val Leu Asp Gln Gly Glu Leu Ser Tyr Leu His Gly
            20              25              30
```

46

```
His Arg Asn Leu Ile Pro Gly Leu Glu Glu Ala Leu Glu Gly Arg Glu
        35                  40                  45

Glu Gly Glu Ala Phe Gln Ala His Val Pro Ala Glu Lys Ala Tyr Gly
        50                  55                  60

Ala Gly Ser Gly Gly Gly Gly Tyr Arg Tyr Arg Gln Gly Gly Gly Gly
65                  70                  75                  80

Gly Ser Ser Gly Lys Asp Leu Asp Phe Gln Val Glu Val Val Lys Val
                85                  90                  95

Arg Glu Ala Thr Pro Glu Glu Leu Leu His Gly His Ala His Gly Gly
                100                 105                 110

Gly Ser Gly Gly Gly Ser Gly Gly Gly Ser Gly Gly Gly Ser Gly Gly
                115                 120                 125

Gly Ser Gly Gly Gly Met Ser Glu Ser Val Gln Ser Asn Ser Ala Val
                130                 135                 140

Leu Val His Phe Thr Leu Lys Leu Asp Asp Gly Thr Thr Ala Glu Ser
145                 150                 155                 160

Thr Arg Asn Asn Gly Lys Pro Ala Leu Phe Arg Leu Gly Asp Ala Ser
                165                 170                 175

Leu Ser Glu Gly Leu Glu Gln His Leu Leu Gly Leu Lys Val Gly Asp
                180                 185                 190

Lys Thr Thr Phe Ser Leu Glu Pro Asp Ala Ala Phe Gly Val Pro Ser
                195                 200                 205

Pro Asp Leu Ile Gln Tyr Phe Ser Arg Arg Glu Phe Met Asp Ala Gly
210                 215                 220

Glu Pro Glu Ile Gly Ala Ile Met Leu Phe Thr Ala Met Asp Gly Ser
225                 230                 235                 240

Glu Met Pro Gly Val Ile Arg Glu Ile Asn Gly Asp Ser Ile Thr Val
                245                 250                 255

Asp Phe Asn His Pro Leu Ala Gly Gln Thr Val His Phe Asp Ile Glu
                260                 265                 270

Val Leu Glu Ile Asp Pro Ala Leu Glu Ala Gly Gly Gly Ser Gly Gly
                275                 280                 285
```

```
        Gly Ser Gly Gly Gly Ser Gly Gly Gly Ser Gly Gly Gly Ser Gly Gly
            290             295             300

        Gly Ile Glu Gly Arg Met Ser Leu Ala Ser Gly Lys Ser Leu Leu His
            305             310             315             320

        Glu Val Asp Lys Asp Ile Ser Gln Leu Thr Gln Ala Ile Val Lys Asn
                        325             330             335

        His Lys Asn Leu Leu Lys Ile Ala Gln Tyr Ala Ala Gln Asn Arg Arg
                    340             345             350

        Gly Leu Asp Leu Leu Phe Trp Glu Gln Gly Gly Leu Ala Lys Ala Leu
                    355             360             365

        Gln Glu Gln Ala Ala Phe Leu Asn Ile Thr Asn Ser His Val Ser Ile
            370             375             380

        Leu Gln Glu Arg Pro Pro Leu Glu Asn Arg Val Leu Thr Gly Trp Gly
            385             390             395             400

        Leu Asn Trp Asp Leu Gly Leu Ser Gln Trp Ala Arg Glu Ala Leu Gln
                        405             410             415

        Thr Gly Gly His His His His His His His
                    420             425
```

<210> 63
<211> 24
<212> PRT
<213> Artificial Sequence

<220>
<223> tryptic peptide ‚62-85'

<400> 63

```
        Ala Tyr Gly Ala Gly Ser Gly Gly Gly Gly Asp Tyr Ala Leu Gln Gly
        1               5               10              15

        Gly Gly Gly Gly Ser Ser Gly Lys
                    20
```

<210> 64
<211> 28
<212> PRT
<213> Artificial Sequence

<220>
<223> tryptic peptide ‚241-255'

<400> 64

| Ala | Leu | Gln | Glu | Gln | Ala | Ala | Phe | Leu | Asn | Ile | Thr | Asn | Ser | His | Val |
|-----|-----|-----|-----|-----|-----|-----|-----|-----|-----|-----|-----|-----|-----|-----|-----|
| 1 | | | | 5 | | | | | 10 | | | | | 15 | |

| Ser | Ile | Leu | Gln | Glu | Arg | Pro | Pro | Leu | Glu | Asn | Arg |
|-----|-----|-----|-----|-----|-----|-----|-----|-----|-----|-----|-----|
| | | | 20 | | | | | 25 | | | |

<210> 65
<211> 15
<212> PRT
<213> Artificial Sequence

<220>
<223> tryptic peptide ‚241-255'

<400> 65

| Glu | Ala | Leu | Gln | Thr | Gly | Gly | His | His | His | His | His | His | His | His |
|-----|-----|-----|-----|-----|-----|-----|-----|-----|-----|-----|-----|-----|-----|-----|
| 1 | | | | 5 | | | | | 10 | | | | | 15 |

**Description**

The present invention relates to a recombinant transglutaminase (TG) substrate comprising an amino acid sequence of the FKBP domain of an FKBP polypeptide, wherein the "insert-in-flap" (IF) domain thereof is, at least in part, replaced by an amino acid sequence ("Q-tag") of 5 to 15 amino acids comprising a sequence having at least 80% sequence identity to the peptide sequence $X_1$-YRYRQ-$X_2$ (SEQ ID NO. 1), specifically to the YRYRQ portion of the peptide sequence, and wherein said TG substrate is a substrate for the TG function of the *Kutzneria albida* TG (KalbTG). The present invention furthermore relates uses of said substrate.

**Background of the invention**

Soluble and/or immunoreactive antigens and variants thereof are essential for *in vitro* diagnostic tests. As an example, a recombinant and soluble variant of the viral coat protein gp41 is used in order to detect an HIV-1 infection.
The required solubility of immunoreactive antigens and variants thereof can be a challenge in the design of effective assays, but can be improved by fusing to one or more chaperone units having peptidyl prolyl isomerase (PPIase) activity. The technique of using a protein scaffold for engineering polypeptide domains displayed by the scaffold is known in the field of antibodies and antibody fragments. Thus, domains such as variable loops of antigen binding regions of antibodies have been extensively engineered to produce amino acid sequence segments having improved binding (e.g. affinity and/or specificity) to known targets (see WO 2014/071978).
FK506 binding proteins (FKBPs) have been identified in many eukaryotes from yeast to humans and function as protein folding chaperones for proteins containing proline residues. Along with cyclophilin, FKBPs belong to the immunophilin family. In the human genome there are encoded fifteen proteins whose segments have significant homology with the sequence of 12 kDa protein which is the target of the potent immunosuppressive macrolides FK506 or rapamycin. The 12 kDa archetype of the FK506-binding protein (FKBP), known as FKBP-12, is an abundant intracellular protein. FKBP12 functions as a PPIase that catalyzes interconversion between prolyl cis/trans conformations. FKBPs are involved in diverse cellular functions including protein folding, cellular signaling, apoptosis and transcription. They elicit their function through direct binding and altering conformation of their target proteins, hence acting as molecular switches.
The bacterial slyD gene encodes a FKBP-type peptidyl-prolyl cis-trans isomerase (PPIase). SlyD is a bacterial two-domain protein that functions as a molecular chaperone, a prolyl cis/trans isomerase, and a nickel-binding protein. The chaperone function located in one domain of SlyD is involved in twin-arginine translocation and increases the catalytic efficiency of the prolyl cis/trans isomerase domain in protein folding by two orders of magnitude.
Schlapschy and Skerra (in: Periplasmic chaperones used to enhance functional secretion of proteins in *E. coli*. Methods Mol Biol. 2011;705:211-24) disclose that problems with the folding of the recombinant gene product as well as protein aggregation, i.e., formation of inclusion bodies, are frequently encountered in Escherichia coli. This is particularly true for proteins that carry structural disulfide bonds, including antibody fragments, cytokines, growth factors, and extracellular fragments of eukaryotic cell surface receptors. Therefore, they have developed the helper plasmid pTUM4, which effects overexpression of four established periplasmic chaperones and/or folding catalysts: the thiol-disulfide oxidoreductases DsbA and DsbC, which catalyze the formation and isomerization of disulfide bridges, and two peptidyl-prolyl cis/trans isomerases with chaperone activity, FkpA and SurA.
The *E. coli* SlyD and FKBP12 (wild type and mutants C23A and C23S) can be recombinantly produced in *E. coli* in high yield in soluble form (Standaert, R.F., et al, Nature 346 (1990) 671-674). FKBP derived from thermophilic organisms and E. coli SlyD can be used as chaperones in the recombinant expression of chimeric polypeptides in E. coli (Ideno, A., et al, Appl. Microbiol. Biotechnol. 64 (2004) 99-105). The E. coli SlyD and FKBP12 polypeptides are reversibly folding polypeptides (Scholz, C, et al, J. Biol. Chem. 271 (1996) 12703-12707). Low C, et al. J. Mol. Biol. 398 (2010) 375-390 report the crystal structure and functional characterization of the metallochaperone SlyD from Thermus thermophilus. Thermus thermophilus consists of two domains representing two functional units. PPIase activity is located in a typical FKBP domain, whereas chaperone function is associated with the autonomously folded insert-in-flap (IF) domain. The two isolated domains are stable and functional in solution.
Kovermann et al (in: Kovermann M, Schmid FX, Balbach J Molecular function of the prolyl cis/trans isomerase and metallochaperone SlyD. Biol Chem. 2013 Aug;394(8):965-75) disclose that SlyD is a bacterial two-domain protein that functions as a molecular chaperone, a prolyl cis/trans isomerase, and a nickel-binding protein. They summarize recent findings about the molecular enzyme mechanism of SlyD. The chaperone function located in one domain of SlyD is involved in twin-arginine translocation and increases the catalytic efficiency of the prolyl cis/trans isomerase domain in protein folding by two orders of magnitude.
The amino acid sequence of the FKBP 12 polypeptide comprises a single tryptophan residue at position 60. Thus, FKBP12 mutants can be analyzed for structural integrity simply by analyzing the tryptophan fluorescence (De-Cenzo, M.T., et al, Protein Eng. 9 (1996) 173-180). A test for remaining catalytic activity of the FKBP 12 mutant can be performed by determining the remaining rotamase activity (Brecht, S., et al., Neuroscience 120 (2003) 1037-1048;

Schories, B., et al, J. Pept. Sci. 13 (2007) 475-480; Timerman, A.P., et al, J. Biol. Chem. 270 (1995) 2451-2459). It is also possible to determine the structural integrity of FKBP 12 mutants by determining the FK506- or Rapamycin binding (DeCenzo, M.T., et al, Protein Eng. 9 (1996) 173-180).

Geitner et al. (in: Geitner AJ1, Varga E, Wehmer M, Schmid FX. Generation of a highly active folding enzyme by combining a parvulin-type prolyl isomerase from SurA with an unrelated chaperone domain. J Mol Biol. 2013 Nov 15;425(22):4089-98) disclose parvulins as small prolyl isomerases that serve as catalytic domains of folding enzymes. SurA (survival protein A) from the periplasm of *Escherichia coli* consists of an inactive (Par1) and an active (Par2) parvulin domain as well as a chaperone domain. In the absence of the chaperone domain, the folding activity of Par2 is virtually abolished. They created a chimeric protein by inserting the chaperone domain of SlyD, an unrelated folding enzyme from the FKBP family, into a loop of the isolated Par2 domain of SurA. This increased its folding activity 450-fold to a value higher than the activity of SurA, in which Par2 is linked with its natural chaperone domain. In the presence of both the natural and the foreign chaperone domain, the folding activity of Par2 was 1500-fold increased. Related and unrelated chaperone domains thus are similarly efficient in enhancing the folding activity of the prolyl isomerase Par2. A sequence analysis of various chaperone domains suggests that clusters of exposed methionine residues in mobile chain regions might be important for a generic interaction with unfolded protein chains. This binding is highly dynamic to allow frequent transfer of folding protein chains between chaperone and catalytic domains.

For immunological diagnostic systems, antigens and/or antibodies or other immunological binding partners (proteins of interest) are furthermore often conjugated, for example with biotin (for an immobilization with streptavidin) or other labels, like ruthenium, for their detection. Usually, chemical methods are used in order to conjugate such markers to reactive amino- or sulfhydryl-moieties.

Unfortunately, conventional chemical strategies for protein modification are difficult to control and give rise to heterogeneous populations of immunoconjugates with variable stoichiometries, each of which has its own in vivo characteristics. Furthermore, the number of conjugated molecules or label can not be controlled, and thus is not defined, and usually follows a normal distribution, which causes problems when wishing to quantify reactions.

The introduction of artificial, bio-orthogonal groups for site-specific and stoichiometric protein modification offers a potential solution to this problem. Such strategies are en vogue but are often laborious and still risk product heterogeneity. Furthermore, all components of the system must be and have to remain stable over prolonged periods of time, and under conjugation conditions. Also, the position/location of the conjugation(s) can not be controlled. A nonspecific inclusion of the marker, for example in or close to the active center of a protein, or at a position at or close to the position(s) that mediate immunological activities can interfere with the immune-reactivity or even fully inhibit it.

Methods for a site-specific enzymatic conjugation of markers are known (e.g. sortase, MTG), but these require the presence of an additional specific recognition sequence (tag sequence) in the protein of interest to be labelled

Microbial transglutaminase (MTG) is one of the most important enzymes for the crosslinking of proteins and peptides in many food- and biotechnological applications. MTG was first discovered in and later extracted from the organism *Streptomyces mobaraensis.* Recombinantly produced *Streptomyces mobaraensis* MTG represents the bulk of industrially used MTG today. The enzyme catalyzes the formation of an isopeptide bond between an acyl-group, e.g. a glutamine (Q) side chain and an alkyl-amine, e.g. a lysine (K) side chain. In absence of reactive amine groups, the enzymatic reaction with water leads to deamination of Glutamine side chains. The bacterial enzyme works without the addition of cofactors such as $Ca^{2+}$ or GTP and in a broad range of pH-, buffer- and temperature conditions. MTG has already been used for the development of therapeutic Antibody-drug conjugates, but due to the low specificity of the enzyme, the large-scale production of such MTG-mediated conjugates has not yet been established. All known active microbial transglutaminase species exhibit molecular weights > 38 kDa. Being a cross-linking enzyme in nature, microbial transglutaminase displays broad substrate specificity for amine-donor molecules and a relatively low specificity for acyl-donors. Since only the substrate specificities of the enzyme from *Streptomyces mobaraenis* and homolog enzymes are known, a bio-orthogonal conjugation approach, e.g. simultaneous labeling of a biomolecule using two or more different label-substrates and two or more transglutaminase species, is currently not possible.

WO 00/43492 and WO 2012/059882 disclose a recombinant TG substrate that is adapted for a different transglutaminase than in the present invention. Furthermore, the presentation of the Q-tag embedded in the FKBP domain according to the invention does not substantially interfere with any other functional component of the protein of interest.

In view of the above, it is an object of the present invention to provide new tools and methods in order to provide for an efficient and controlled labelling of antigens and/or antibodies or other immunological binding partners (proteins of interest) in the context of immunological diagnostic and/or therapeutic systems and methods. Other aspects and objects will become apparent for the person of skill upon reading the following more detailed description of the invention.

According to a first aspect thereof, the above objects are solved by the provision of a recombinant transglutaminase (TG) substrate according to the following general formula I

$$(F^*\text{-}L)_y\text{-}X \qquad (I).$$

In said formula (I), F* is selected from an amino acid sequence of the FKBP domain which comprises the N-terminal amino acids 1 to 64 and 123 to 149 of the SLYD polypeptide , wherein amino acids 65 to 122 are replaced by an amino acid sequence ("Q-tag") of 5 to 15 amino acids, the Q-tag comprising a sub-sequence of 5 contiguous amino acids having at least 80% sequence identity to the YRYRQ portion of the peptide sequence $X_1$-YRYRQ-$X_2$ (SEQ ID NO. 1), wherein $X_1$ and $X_2$ are absent or constitute linker amino acids, wherein the Q-tag is an amino acid sequence selected from the group consisting of SEQ ID NO:8, SEQ ID NO:11, SEQ ID NO:18 and SEQ ID NO:20; L is absent or is selected from a linker amino acid sequence; and X is a protein of interest selected from an enzyme, an antigen, such as a viral protein, an antibody or fragment thereof, and other immunological binding partners; y is an integer of between 1 and 100, and wherein said TG substrate is a substrate for the TG function of the *Kutzneria albida* TG according to SEQ ID No. 23.

It is further understood that, according to the invention, the linker amino acids $X_1$ and $X_2$, if present, are selected independently from each other.

Preferred are Q-tags according to the present invention that a re selected from the sequences $X_1$-YKYRQ-$X_2$ (SEQ ID NO: 8), $X_1$-LRYRQ-$X_2$ (SEQ ID NO: 11), $X_1$-PRYRQ-$X_2$ (SEQ ID NO: 18), and $X_1$-VRYRQ-$X_2$ (SEQ ID NO: 20), wherein $X_1$ and $X_2$ are as above.

It was surprisingly found that the two parts of the FKBP domain upstream and downstream insertion of the IF domain function as structurally rather rigid and precise scaffold or "collar" for the sequence that is inserted into and/or replaces the IF domain part ("head", "Q-tag"). Because of this, the presentation and orientation of the insertion/replacement reliably does not substantially interfere with any other function of the other components of the substrate, in particular the function(s) of the protein of interest (X). Furthermore, the presentation of the - in this case - TG substrate binding site (Q-tag) leads to a highly controlled stoichiometric binding of the label, and hence labelling of the protein of interest.

In formula (I), L is absent or is selected from a linker amino acid sequence. Preferably, said linker sequence L comprises between 1 to 20 amino acids and more preferred said amino acids do not interfere essentially with the function(s) of the FKBP domain (in particular the replacement/insertion) and/or the protein(s) of interest (e.g. immunological functions).

X designates the protein of interest; that is preferably selected from an enzyme, an antigen, such as a viral protein, an antibody or fragment thereof, and other immunological binding partners.

In formula (I), y is an integer of between 1 and 100, thus, several marker groups F*-L can be attached to the protein of interest.

Preferably, the recombinant transglutaminase (TG) substrate according to the invention can furthermore comprise protein tags for purification and/or immobilization, for example at the N- and/or C-terminus, like biotin, maltose or his-tags(s).

Preferably, the different components of the substrate F*, L and/or X are covalently attached with each other in order to allow the controlled labeling of the substrate using the TG activity. The substrate can be recombinantly produced as a fusion protein, and expressed and purified from hosts cells, such as, for example, bacterial or yeast host cells. Respective methods are well known to the person of skill. The components can also be produced (e.g. synthesized) separately or in parts, and are then subsequently joined, either covalently or conjugated, depending on the circumstances and the desired purpose(s) thereof.

According to the invention, the inventive TG substrate is a substrate for the transglutaminase (TG) function of the *Kutzneria albida* TG according to SEQ ID No. 23, or a homologous protein that is identical to at least 80%, preferably to at least 90%, more preferably to at least 95, 98 or 99% to the amino acid sequence thereof, and exhibit substantial TG activity as described herein. Preferably, the TG polypeptide or a part thereof having a substantial transglutaminase function or activity is cloned and recombinantly produced in hosts cells, and subsequently (at least in part) purified, depending on the circumstances and the desired purpose(s) thereof. Respective methods are well known to the person of skill. The TG activity can be measured using assays that are also well known to the person of skill. "Recombinant TG substrate" in the context of the invention shall mean that the substrate as a whole does not occur in nature.

The FKBP domain as used for the recombinant transglutaminase (TG) substrate according to the present invention is preferably selected from a eukaryotic or bacterial FKBP polypeptide selected from FKBP12, AIP, AIPL1, FKBP1A, FKBP1B, FKBP2, FKBP3, FKBP5, FKBP6, FKBP7, FKBP8, FKBP9, FKBP9L, FKBP10, FKBP11, FKBP14, FKBP15, FKBP52, LOC541473, and SLYD, and homologs of the FKBP domains thereof that are identical to at least 80%, preferably to at least 90%, more preferably to at least 95, 98 or 99% to the amino acid sequence thereof, and are suitable for the insertion of a Q-tag. Respective domains can be analyzed for their suitability as described herein, and in the literature, e.g. using alignment programs, in particular to identify structural alignments.

In an embodiment, a FKBP domain as used for the recombinant transglutaminase (TG) substrate according to the present invention comprises an amino acid sequence of a polypeptide with PPIase activity. The polypeptide with PPIase activity is of prokaryotic or eukaryotic origin, or the polypeptide with PPIase activity as an artificial variant (mutant) thereof. An artificial variant of a polypeptide with PPIase activity can be generated by a technique according to the state of the art of protein engineering. According to the invention, an artificial variant of a polypeptide with PPIase activity is characterized e.g. by replacement, addition or deletion of one or more amino acids. In a specifc embodiment, PPIase activity and/or chaperone function of the FKBP domain as used for the recombinant transglutaminase substrate according to the present invention is/are preserved in an artificial variant.

Knappe et al. (in: Knappe TA, Eckert B, Schaarschmidt P, Scholz C, Schmid FX. Insertion of a chaperone domain converts FKBP12 into a powerful catalyst of protein folding. J Mol Biol. 2007 May 18;368(5):1458-68. Epub 2007 Mar 14) disclose that the catalytic activity of human FKBP12 as a prolyl isomerase is high towards short peptides, but very low in proline-limited protein folding reactions. In contrast, the SlyD proteins, which are members of the FKBP family, are highly active as folding enzymes. They contain an extra "insert-in-flap" or IF domain near the prolyl isomerase active site. The excision of this domain did not affect the prolyl isomerase activity of SlyD from *Escherichia coli* towards short peptide substrates but abolished its catalytic activity in proline-limited protein folding reactions. The reciprocal insertion of the IF domain of SlyD into human FKBP12 increased its folding activity 200-fold and generated a folding catalyst that is more active than SlyD itself. The IF domain binds to refolding protein chains and thus functions as a chaperone module.

In *E. coli,* it is believed that amino acids 1 to 69 of SLYD form the first part of the PPIase domain, amino acids 76 to 120 form the IF-chaperone (domain), and amino acids 129 to 151 form the second part of the PPIase domain. Thus, amino acids 1 to 151 form the FKBP-domain. Amino acids 152 to 196 are involved in metal binding.

Preferred is the recombinant transglutaminase (TG) substrate according to the present invention, wherein said FKBP domain has a length of between about 120 to 170, preferably between about 130 to 160, and most preferred between about 145 to 155 of the N-terminal amino acids of said FKBP polypeptide. This includes the IF-domain sequence.

"About" in the context of the present invention shall mean +/- 10% of a given value.

Preferred is the recombinant transglutaminase (TG) substrate according to the present invention wherein said FKBP domain comprises the N-terminal amino acids 1 to 64 and 123 to 149 of the SLYD polypeptide, and wherein amino acids 65 to 122 are replaced by said Q-tag.

As mentioned above, the linker sequence L can comprise between 1 to 20 amino acids, preferably 1 to 10 amino acids, more preferred 1 to 5 amino acids wherein preferably said amino acids to not interfere essentially with the FKBP domain and/or the protein of interest. Preferred linker amino acids are small amino acids, like glycine or alanine. Amino acid linkers and their compositions are known in the art (see, e.g. Chichili et al. Linkers in the structural biology of protein-protein interactions Protein Sci. 2013 Feb; 22(2): 153-167).

Proteins of interest in the context of the present invention are generally all proteins that can be labelled using the present technology. Preferred is the recombinant transglutaminase (TG) substrate according to the present invention, wherein said protein of interest is selected from an enzyme, an antigen, such as a viral protein, an antibody or fragment thereof, and other immunological binding partners. The present invention is of particular use for immunological reactions and assays, where quantification is desired.

Another aspect of the present invention then relates to an in vitro method for labelling a protein of interest, comprising a) providing the recombinant transglutaminase (TG) substrate according to the present invention comprising a protein of interest; b) providing an effective amount of the transglutaminase of *Kutzneria albida* (e.g. according to SEQ ID NO: 23) or a homolog thereof as described herein, c) providing a suitable label comprising an alkyl-amine group ("amine donor"), such as, for example, a lysine, and d) contacting said components according to a) to c), whereby said transglutaminase (activity) attaches said label to said substrate.

Preferred is a method according to the present invention, wherein said transglutaminase of *Kutzneria albida* or a homolog thereof as described herein is recombinantly produced, as described herein.

In this method according to the present invention, the substrate comprising the F* group comprising the Q-tag (optionally replacing the IF-domain region) is labelled using the activity of the transglutaminase of *Kutzneria albida*. Preferably, the label to be attached is selected from an enzyme, biotin, a radioactive group, a dye, such as a fluorescent dye, an isotope, and a metal. Said label is part of the label component/compound that comprises alkyl-amine group, such as, for example, a lysine. Most preferred, said label component/compound comprises an amine-donor tag ("K-tag") having at least 80% sequence identity to the peptide sequence RYESK. Examples for preferred K-tags and their composition are described below and can also be found in the literature.

Preferred is a method according to the present invention, wherein said labeling is controlled and, for example, achieved in a stoichiometric ratio of label and protein of interest, for example at about 1:1. Multiple labeling can also be achieved using additional enzymes (e.g. other TGs than Kalb-TG) and respective other TG-substrates, in order to attach two or even multiple labels to a protein/proteins of interest.

Preferred is the method according to the present invention, wherein said protein of interest is selected from an enzyme, an antigen, such as a viral protein, an antibody or fragment thereof, and other immunological binding partners. The present invention is of particular use for immunological reactions and assays, where quantification is desired.

The choice of the protein of interest can also depend on the intended use of the piRNA molecules as used, either in therapy, research and/or diagnosis. Preferred is the method according to the invention, wherein said protein of interest is selected from protein involved/causing in cancer, neurological diseases, immunological diseases, allergy, metabolic diseases, fertility (e.g. reproductive rate or number), animal production (e.g. amount of meat or milk), protein essential for cell growth and/or development, for mRNA degradation, for translational repression, and/or for transcriptional gene silencing.

Another aspect of the present invention then relates to a pharmaceutical or diagnostic composition comprising at least one recombinant transglutaminase (TG) substrate according to the invention, together with pharmaceutically acceptable carriers and components, such as buffers. Pharmaceutical or diagnostic compositions for multiple labeling can also be obtained that comprise additional enzymes (e.g. other TGs than Kalb-TG) and respective other TG-substrates, in order to be able to attach two or even multiple labels to a protein/proteins of interest.

Another aspect of the present invention then relates to a pharmaceutical or diagnostic composition comprising at least one labeled protein of interest as produced according to a method according to the present invention, together with pharmaceutically acceptable carriers and components, such as buffers.

Preferred is the pharmaceutical or diagnostic composition according to the present invention, wherein said protein of interest is labelled at a stoichiometric ratio of label and protein of interest of, for example, about 1:1. Two or even several labels can also be attached to a protein/proteins of interest.

Another aspect of the present invention then relates to a diagnostic kit, comprising the diagnostic composition according to the present invention, optionally together with other components for performing an immunoassay. The kit can comprise, in joint or separate containers, a microbial recombinant transglutaminase, e.g. a purified microbial transglutaminase having at least 80% sequence identity to the *Kutzneria albida* microbial transglutaminase as described herein. The kit may further include substrates, such as a substrate including an amine-donor tag having at least 80% sequence identity to the peptide sequence RYESK. The kit can also include instructions for performing reactions (e.g. immunoassays) that require use of the substrate according to the invention, either labeled or un-labeled.

Disclosed is the use of the recombinant transglutaminase (TG) substrate, the pharmaceutical or diagnostic composition or the kit according to the present invention for labeling or in the labeling of a protein of interest, in particular in immunological reactions and assays, where a quantification is desired.

The methods of the present invention can be performed in vivo or in vitro, in laboratory animals, or in culture.

The present invention will now be illustrated further in the following non-limiting examples, with reference to the accompanying figures.

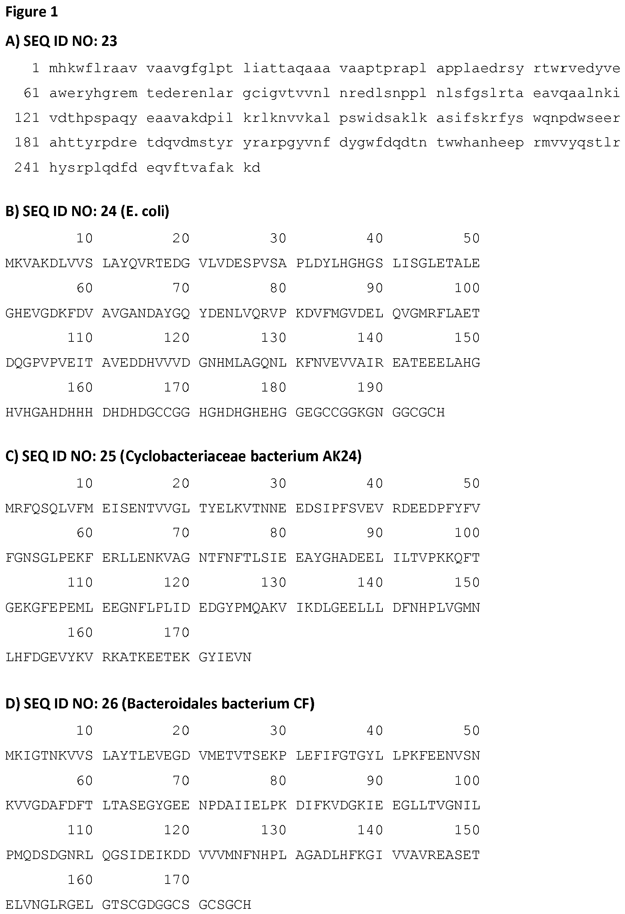

Figure 1 shows the amino acid sequences of the transglutaminase from *Kutzneria albida* (database no. WP_030111976), the amino acid sequence of slyD of *E. coli,* the amino acid sequence of slyD of *Cyclobacteriaceae bacterium* AK24, and the amino acid sequence of slyD of *Bacteroidales bacterium.*

Figure 2 shows the results of labeling using Cy3 or Cy5 according to the examples, below.

Figure 2B shows the relative pH independence in the range of between 6.2 to 9.0.

Figure 3A shows the general structure of a rhutenium labelled fusion construct, and Figure 3A shows the results of labeling using SDS-PAGE according to the examples, below.

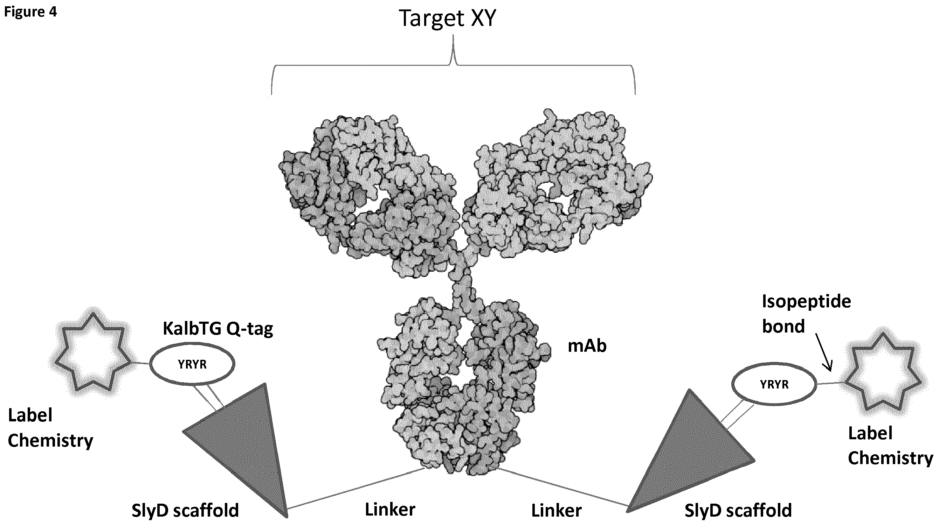

Figure 4 shows a preferred embodiment of a substrate-labeled protein of interest (here, two chains of an antibody) according to the present invention.

Figure 5 A presents a schematic view of 'TtSlyQD-Xa-gp21-8H' of SEQ ID NO:60. The SlyD portions are indicated, further the Q-tag in the fusion protein, as well as a recognition site for factor Xa protease, and the 'gp21' portion as indicated in the examples. The star symbolizes a Ruthenium label.

Figure 5 B shows results after SDS-PAGE analysis showing specific and unspecific Ruthenium labeling of the recombinant gp21 (HTLV) antigen with the bacterial transglutaminase of *Streptomyces mobaraensis.* Depicted are unlabeled, single-labeled, double-labeled, and triple-labeled 'TtSlyQD-Xa-gp21-8H' fusion proteins after Ru labeling.

Figure 5 C depicts the Ruthenium label.

Figures 6 A-E present results using labeled 'TtSlyKQD-SlpA-Xa-gp21-8H' according to SEQ ID NO:62 in an Elecsys assay.

SEQ ID NO: 1 to 22 show the sequences of Q-tag motifs as identified, wherein the N-terminal X is as $X_1$ above, and the C-terminal X is as $X_2$ as above.

SEQ ID NO: 23 shows the amino acid sequence of the transglutaminase from *Kutzneria albida* (database no. WP_030111976).

SEQ ID NO: 24 shows the amino acid sequence of slyD of *E. coli.*

SEQ ID NO: 25 shows the amino acid sequence of slyD of *Cyclobacteriaceae bacterium* AK24.

SEQ ID NO: 26 shows the amino acid sequence of slyD of *Bacteroidales bacterium* CF.

SEQ ID NO: 27 shows the amino acid sequence of the FKBP domain of slyD of *E. coli.*

SEQ ID NO: 28 shows the amino acid sequence of the FKBP domain of FKBP16 of *E. coli.*

SEQ ID NO: 29 shows the amino acid sequence of the FKBP domain of slyD of *Thermus thermophilus.*

SEQ ID NO: 30 to 51 show the sequences of Q-tag motifs as identified, wherein the N-terminal and C-terminal amino acid is one exemplary glycine linker.

SEQ ID NO: 52 shows the amino acid sequence of the KalbTG glutamine donor sequence that was recombinantly grafted onto the FKBP domain of SlyD.

SEQ ID NO: 53 shows the amino acid sequence of a transglutaminase lysine donor sequence (K-tag).

SEQ ID NO: 54 shows the SlyD amino acid sequence.

SEQ ID NO: 55 shows the amino acid sequence of SlyD with a MTG Q-tag.

SEQ ID NO: 56 shows the amino acid sequence of SlyD with a KalbTG Q-tag.

SEQ ID NO: 57 shows the SlpA amino acid sequence.

SEQ ID NO: 58 shows the main HTLV antigen and viral envelope glycoprotein amino acid sequence 'gp21'.

SEQ ID NO: 59 shows the amino acid sequence of modified the 'gp21' ectodomain polypeptide sequence, engineered for better solubility, stability and reactivity in the immunoassay.

SEQ ID NO: 60 shows the amino acid sequence of the recombinant fusion protein 'TtSlyQD-Xa-gp21-8H'.

SEQ ID NO: 61 shows the amino acid sequence of the recombinant fusion protein 'TtSlyD-Xa-gp21-8H'.

SEQ ID NO: 62 shows the amino acid sequence of the recombinant fusion protein 'TtSlyKQD-SlpA-Xa-gp21-8H'.

## EXAMPLES

### Identification of the position of the IF domain

In principle, alignments of the primary structure of the amino acid sequences of FKBP domains can be used in order to identify the position of the IF domain to be replaced (or added), e.g. using the program Clustal Omega. An alternative, in particular with respect to non-bacterial FKBPs is the alignment of 3D structures (e.g. using the program PyMol), since the domain constitutes a structurally conserved element.
Specific examples are as follows:

SLYD_ECOLI FKBP-type peptidyl-prolyl cis-trans isomerase SlyD of Escherichia coli (strain K12). FKBP domain, IF domain double underlined.

MKVAKDLVVSLAYQVRTEDGVLVDESPVSAPLDYLHGHGSLISGLETALEGHEVGDKFDVAV
GANDAYG<u>QYDENLVQRVPKDVFMGVDELQVGMRFLAETDQGPVPVEITAVEDDHVVVDGNHM</u>
<u>LA</u>GQNLKFNVEVVAIREATEEELAHGHVHGAHDHHHDHDHD  (SEQ ID NO: 27).

ECOLI FKBP-type 16 kDa peptidyl-prolyl cis-trans isomerase of Escherichia coli (strain K12). FKBP domain, IF domain double underlined.

MSESVQSNSAVLVHFTLKLDDGTTAESTRNNGKPALFRLGDASLSEGLEQHLLGLKVGDKTT
FSLEPDAAFG<u>VPSPDLIQYFSRREFMDAGEPEIGAIMLFTAMDGSEMPGVIREINGDSITVD</u>
<u>FNHPLA</u>GQTVHFDIEVLEIDPALEA  (SEQ ID NO: 28).

Peptidyl-prolyl cis-trans isomerase of Thermus thermophilus (strain HB8/ATCC 27634) FKBP domain, IF domain double underlined.

MKVGQDKVVTIRYTLQVEGEVLDQGELSYLHGHRNLIPGLEEALEGREEGEAFQAHVPAEKA
YG<u>PHDPEGVQVVPLSAFPEDAEVVPGAQFYAQDMEGNPMPLTVVAVEGEEVTVDFNHPLAG</u>K
DLDFQVEVVKVREATPEELLHGHAH  (SEQ ID NO: 29).

**Analysis of the Q-tag sequences for KalbTG**

Analysis of the KalbTG peptide substrates that were identified using labeling assays with a set of peptides comprising 5-mer amino acid sequences with three 3 N- and C-terminal glycine residues ($G_1$ and $G_2$) attached revealed a set of characteristics shared by these substrates. Specific examples were as follows:

$G_1$-YRYRQ-$G_2$ (SEQ ID NO: 30), $G_1$-RYRQR-$G_2$ (SEQ ID NO: 31), $G_1$-RYSQR-$G_2$ (SEQ ID NO: 32), $G_1$-FRQRQ-$G_2$ (SEQ ID NO: 33), $G_1$-RQRQR-$G_2$ (SEQ ID NO: 34), $G_1$-FRQRG-$G_2$ (SEQ ID NO: 35), $G_1$-QRQRQ-$G_2$ (SEQ ID NO: 36), $G_1$-YKYRQ-$G_2$ (SEQ ID NO: 37), $G_1$-QYRQR-$G_2$ (SEQ ID NO: 38), $G_1$-YRQTR-$G_2$ (SEQ ID NO: 39), $G_1$-LRYRQ-$G_2$ (SEQ ID NO: 40), $G_1$-YRQSR-$G_2$ (SEQ ID NO: 41), $G_1$-YQRQR-$G_2$ (SEQ ID NO: 42), $G_1$-RYTQR-$G_2$ (SEQ ID NO: 43), $G_1$-RFSQR-$G_2$ (SEQ ID NO: 44), $G_1$-QRQTR-$G_2$ (SEQ ID NO: 45), $G_1$-WQRQR-$G_2$ (SEQ ID NO: 46), $G_1$-PRYRQ-$G_2$ (SEQ ID NO: 47), $G_1$-AYRQR-$G_2$ (SEQ ID NO: 48), $G_1$-VRYRQ-$G_2$ (SEQ ID NO: 49), $G_1$-VRQRQ-$G_2$ (SEQ ID NO: 50), and $G_1$-YRQRA-$G_2$ (SEQ ID NO: 51).

For the KalbTG, the data revealed that an acyl-donor substrate including a 5-mer amino acid sequence having the formula $Xaa_1$-$Xaa_2$-$Xaa_3$-$Xaa_4$-$Xaa_5$, where Xaa is any amino acid, generally complied with several design rules. First, each 5-mer sequence included at least one glutamine (Q). More particularly, at least one of the third, fourth, and fifth positions of the 5-mer sequence (i.e., $Xaa_3$, $Xaa_4$, and $Xaa_5$) was a glutamine. Several sequences were observed that included a glutamine at each of the third and fifth positions. Furthermore, the observation was made that each 5-mer sequence included at least one arginine (R). More particularly, at least one of the fourth and fifth positions of the 5-mer sequence (i.e., $Xaa_4$ and $Xaa_5$) was an arginine.

**Labeling assays**

**1. Fluorescent dye**

For labeling assays, the chaperone SlyD from *Thermus thermophilus* (Universal Protein Resource (UniProt) Number Q5SLE7) was used as a labeling scaffold for KalbTG. The SlyD sequence is:

MKVGQDKVVTIRYTLQVEGEVLDQGELSYLHGHRNLIPGLEEALEGREEGEAFQAH
VPAEKAYGPHDPEGVQVVPLSAFPEDAEVVPGAQFYAQDMEGNPMPLTVVAVEGEE
VTVDFNHPLAGKDLDFQVEVVKVREATPEELLHGHAH (SEQ ID NO: 29).

A KalbTG glutamine donor sequence (Q-tag, underlined) was recombinantly grafted onto the FKBP domain of SlyD, yielding the following polypeptide sequence:

MKVGQDKVVTIRYTLQVEGEVLDQGELSYLHGHRNLIPGLEEALEGREEGEAFQAH VPAEKAYGAGSGGGGRYRQRGGGGGSSGKDLDFQVEVVKVREATPEELLHGHAHH HHHHHH (SEQ ID NO: 52).

The 8X-histidine-tagged protein was produced in *E. coli* Bl21 Tuner and purified by standard Ni Sepharose-based immobilized metal ion affinity and size exclusion chromatography (HisTrap, Superdex 200; GE Healthcare). All peptides were synthesized via standard Fluorenylmethyloxycarbonyl (FMOC)-based solid phase peptide synthesis.

Labeled peptides were chemically synthesized to have (in order from N-terminus to C-terminus) a Z-protecting group (i.e., a carboxybenzyl group), a transglutaminase lysine donor sequence (K-tag), 8-amino-3,6-dioxaoctanoic acid (O2Oc), peptide, and a Cy3 or Cy5 fluorescent dye. The primary chemical structure of the labeled peptides was:

Z-RYESKG-O2Oc-EUEUEUEUEUEUEUEUEUEUEUEUEUEUEUEUEUEUEU-C(sCy3-MH)-OH (SEQ ID NO: 53).

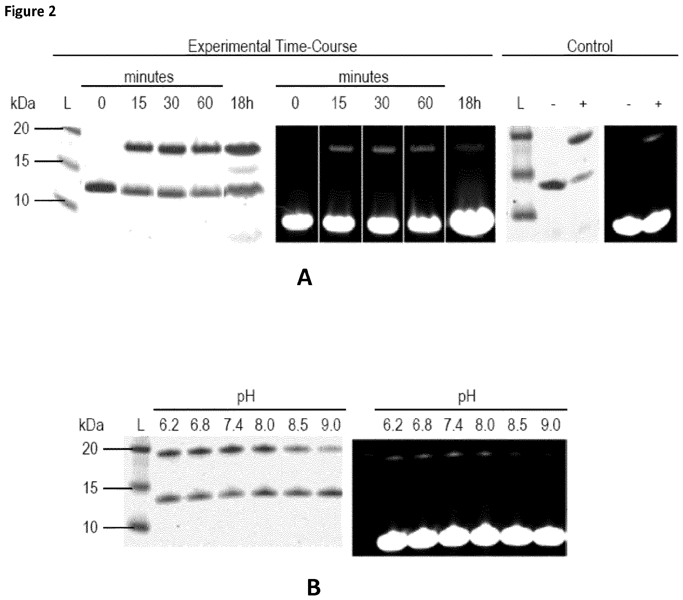

Labeling reactions were performed for 15 minutes at 37 °C in the presence of 72 $\mu$M substrate protein, 720 $\mu$M label peptide and 1 $\mu$M transglutaminase in 200 mM MOPS pH 7.2 and 1 mM EDTA. After incubation for 30 minutes at 37 °C, 1 mM K-tag-Cy5 or -Cy3 was added and incubated for an additional 15 minutes at 37 °C. The reaction was stopped by the addition of 50 mM TCA. Samples were taken between incubation steps and analyzed by SDS-PAGE, in-gel fluorescence (BioRad ChemiDoc gel documentation system, Cy3 and Cy5 LED and filter sets). Results are shown in Figure 2.

**2. Rhutenium label**

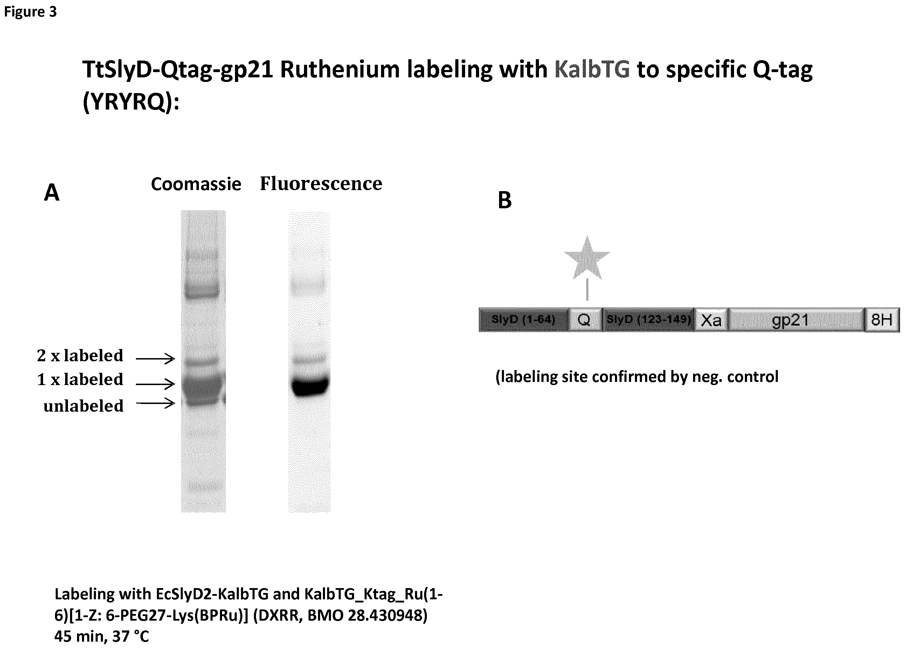

A similar Q-tag construct was tested, consisting of a slyD Q-tag gp21 fusion (see figure 3B), wherein the label was a rhutenium-label. The results in SDS-PAGE in Figure 3A show a good 1:1 ratio of label to construct.

**Production of Biotin- and Ruthenium labeled TtSlyD-gp21 (HTLV) antigens**

For further labeling assays, a recombinant fusion between the chaperone SlyD from *Thermus thermophilus* (Universal Protein Resource (UniProt) Number Q5SLE7), the HTLV viral envelope glycoprotein 'gp21' (Genbank Accession Number DQ224032.1) and, in one example, the chaperone SlpA from *Escherichia coli* (UniProt Number P0AEM0) was used as a labeling scaffold for MTG or KalbTG. The SlyD sequence is:

MKVGQDKVVTIRYTLQVEGEVLDQGELSYLHGHRNLIPGLEEALEGREEGEAFQAH VPAEKAYGPHDPEGVQVVPLSAFPEDAEVVPGAQFYAQDMEGNPMPLTVVAVEGEE VTVDFNHPLAGKDLDFQVEVVKVREATPEELLHGHAH (SEQ ID NO:54)

A MTG or KalbTG glutamine donor sequence (Q-tag) was recombinantly grafted onto the FKBP domain of SlyD, yielding the following polypeptide sequences:

SlyD with MTG Q-tag:

MKVGQDKVVTIRYTLQVEGEVLDQGELSYLHGHRNLIPGLEEALEGREEGEAFQAH VPAEKAYGAGSGGGGDYALQGGGGGSSGKDLDFQVEVVKVREATPEELLHGHAH (SEQ ID NO:55)

SlyD with KalbTG Q-tag:

MKVGQDKVVTIRYTLQVEGEVLDQGELSYLHGHRNLIPGLEEALEGREEGEAFQAH
VPAEKAYGAGSGGGGYRYRQGGGGGSSGKDLDFQVEVVKVREATPEELLHGHAH
(SEQ ID NO:56)

The SlpA sequence is:

MSESVQSNSAVLVHFTLKLDDGTTAESTRNNGKPALFRLGDASLSEGLEQHLLGLKV
GDKTTFSLEPDAAFGVPSPDLIQYFSRREFMDAGEPEIGAIMLFTAMDGSEMPGVIREI
NGDSITVDFNHPLAGQTVHFDIEVLEIDPALEA (SEQ ID NO:57)

The main HTLV antigen and viral envelope glycoprotein sequence 'gp21' is:

IVSSACNNSLILPPFSLSPVPTVGSRSRRAVPVAVWFVSALAMGAGVAGGITGSMSLA
SGKSLLHEVDKDISQLTQAIVKNHKNLLKIAQYAAQNRRGLDLLFWEQGGLCKALQ
EQCCFLNITNSHVSILQERPPLENRVLTGWGLNWDLGLSQWAREALQTGITLVALLLL
VILAGPCIRCPCRTMHP (SEQ ID NO:58)

The gp21 ectodomain polypeptide sequence, engineered for better solubility, stability and reactivity in the immunoassay, is:

MSLASGKSLLHEVDKDISQLTQAIVKNHKNLLKIAQYAAQNRRGLDLLFWEQGGLA
KALQEQAAFLNITNSHVSILQERPPLENRVLTGWGLNWDLGLSQWAREALQTG (SEQ
ID NO:59)

The recombinant fusion sequences used for the labeling assays were:

'TtSlyQD-Xa-gp21-8H' :

MKVGQDKVVTIRYTLQVEGEVLDQGELSYLHGHRNLIPGLEEALEGREEGEAFQAH
VPAEKAYGAGSGGGGDYALQGGGGGSSGKDLDFQVEVVKVREATPEELLHGHAHG
GGSGGGSGGGSGGGSGGGSGGGIEGRMSLASGKSLLHEVDKDISQLTQAIVKNHKNL
LKIAQYAAQNRRGLDLLFWEQGGLAKALQEQAAFLNITNSHVSILQERPPLENRVLT
GWGLNWDLGLSQWAREALQTGGHHHHHHHH (SEQ ID NO:60)

'TtSlyD-Xa-gp21-8H' :

MKVGQDKVVTIRYTLQVEGEVLDQGELSYLHGHRNLIPGLEEALEGREEGEAFQAH

VPAEKAYGPHDPEGVQVVPLSAFPEDAEVVPGAQFYAQDMEGNPMPLTVVAVEGEE

VTVDFNHPLAGKDLDFQVEVVKVREATPEELLHGHAHGGGSGGGSGGGSGGGSGG

GSGGGIEGRMSLASGKSLLHEVDKDISQLTQAIVKNHKNLLKIAQYAAQNRRGLDLL

FWEQGGLAKALQEQAAFLNITNSHVSILQERPPLENRVLTGWGLNWDLGLSQWARE

ALQTGGHHHHHHHH (SEQ ID NO:61)

'TtSlyKQD-SlpA-Xa-gp21-8H':

MKVGQDKVVTIRYTLQVEGEVLDQGELSYLHGHRNLIPGLEEALEGREEGEAFQAH

VPAEKAYGAGSGGGGYRYRQGGGGGSSGKDLDFQVEVVKVREATPEELLHGHAHG

GGSGGGSGGGSGGGSGGGSGGGMSESVQSNSAVLVHFTLKLDDGTTAESTRNNGKP

ALFRLGDASLSEGLEQHLLGLKVGDKTTFSLEPDAAFGVPSPDLIQYFSRREFMDAGE

PEIGAIMLFTAMDGSEMPGVIREINGDSITVDFNHPLAGQTVHFDIEVLEIDPALEAGG

GSGGGSGGGSGGGSGGGSGGGIEGRMSLASGKSLLHEVDKDISQLTQAIVKNHKNLL

KIAQYAAQNRRGLDLLFWEQGGLAKALQEQAAFLNITNSHVSILQERPPLENRVLTG

WGLNWDLGLSQWAREALQTGGHHHHHHHH  (SEQ ID NO:62)

The 8X-histidine-tagged proteins were produced in E. coli B121 Tuner and purified by standard Ni Sepharose-based immobilized metal ion affinity and size exclusion chromatography (HisTrap, Superdex 200; GE Healthcare).
Labeled peptides were chemically synthesized to have (in order from N-terminus to C-terminus) a "Z-" group (i.e., a carboxybenzyl group), a transglutaminase lysine donor sequence (K-tag), polyethylene glycol (PEG), peptide, and a Biotin label or Bipyrimidine Ruthenium (BPRu) complex. The primary chemical structures of the labeled peptides were:
KalbTG K-tag-Bi ("Bi" represents a Biotin label):
Z-RYESKG-PEG27-K(Bi)-OH (2532.0 g/mol)
KalbTG K-tag-Ru ("Ru" represents a Ruthenium-based label for electrochemoluminescence): Z-RYESKG-PEG27-K(BPRu)-OH (2958,4 g/mol)
If not noted otherwise, typical labeling reactions were performed for 15 minutes at 37 °C in the presence of 65 $\mu$M substrate protein, 1000 $\mu$M label peptide and 0.1 $\mu$M transglutaminase in 200 mM MOPS pH 7.4 for labeling with the KalbTG enzyme and at 37 °C in the presence of 72 $\mu$M substrate protein, 720 $\mu$M label peptide and 1 $\mu$M transglutaminase in 200 mM MOPS pH 7.4 with the MTG enzyme. Transglutaminase labeling is described in more detail in applications US 2016/0178627 'System and Method for Identification and Characterization of Transglutaminase Species' and WO 2016/096785 'IDENTIFICATION OF TRANSGLUTAMINASE SUBSTRATES AND USES THEREFOR'.
The labeling reaction mix was separated by size exclusion chromatography (Superdex 200; GE Healthcare) and fractions containing labeled fusion proteins were isolated for subsequent analysis.

**Analysis of Biotin- and Ruthenium labeling and immunoassay**

The quality and quantity of labeling reactions were analyzed by SDS-PAGE. Efficiency of Biotin labeling was estimated by the shift in molecular weight of the bands in the coomassie-stained gel. Efficiency of Ruthenium labeling was estimated by the shift in molecular weight of the bands in the coomassie-stained gel and by in-gel fluorescence (BioRad ChemiDoc gel documentation system, Cy3 LED and filter set). Diagnostic immunoassays (Roche HTLV I/II) were performed according to the manufacturer's instructions on an Elecsys e411 instrument, replacing the biotin labeled antigen moiety in reagent R1 of the test by 'TtSlyD-Xa-gp21-8H' biotin labeled with MTG or 'TtSlyKQD-SlpA-Xa-gp21-8H' biotin labeled with KalbTG, respectively, and replacing the Ruthenium labeled antigen moiety in reagent R2 of the test by 'TtSlyD-Xa-gp21-8H' Ruthenium labeled with MTG or 'TtSlyKQD-SlpA-Xa-gp21-8H' Ruthenium labeled with KalbTG, respectively. Postive controls were performed with HTLV positive sera and the commercially available HTLV I/II kit. Negative controls were performed with HTLV negative sera and with HTLV positive sera in combination with different calibrator and control solutions.

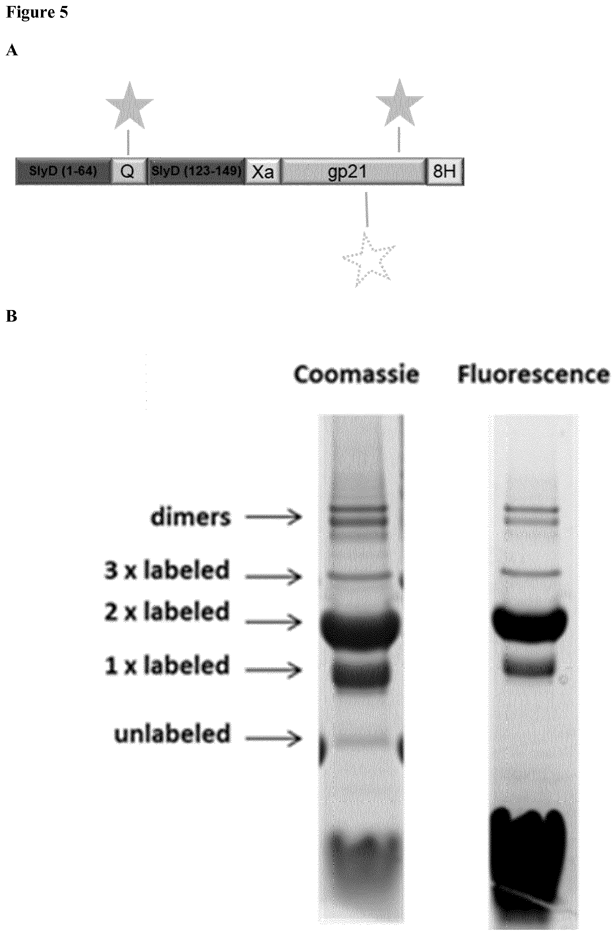

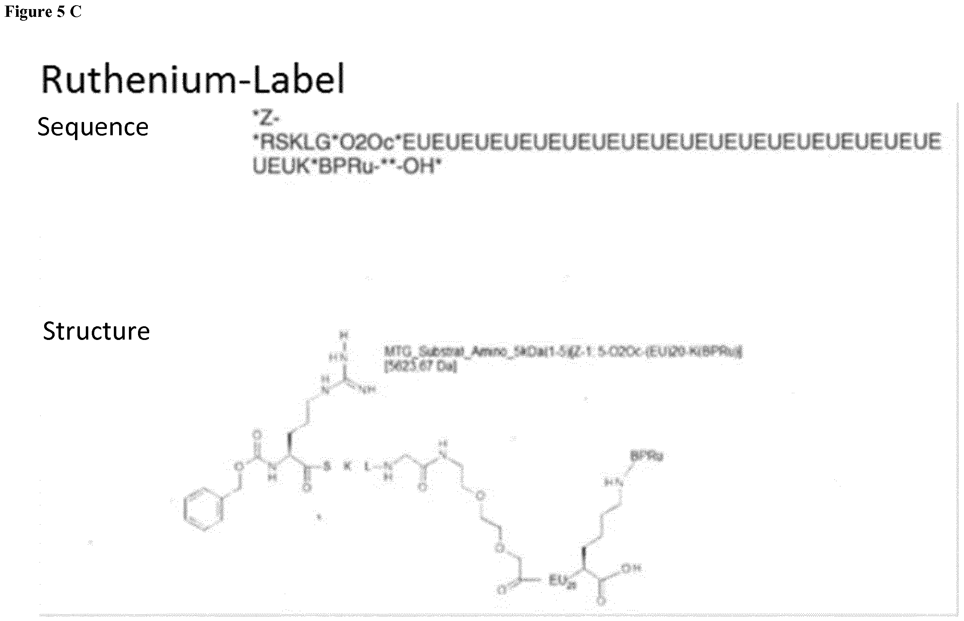

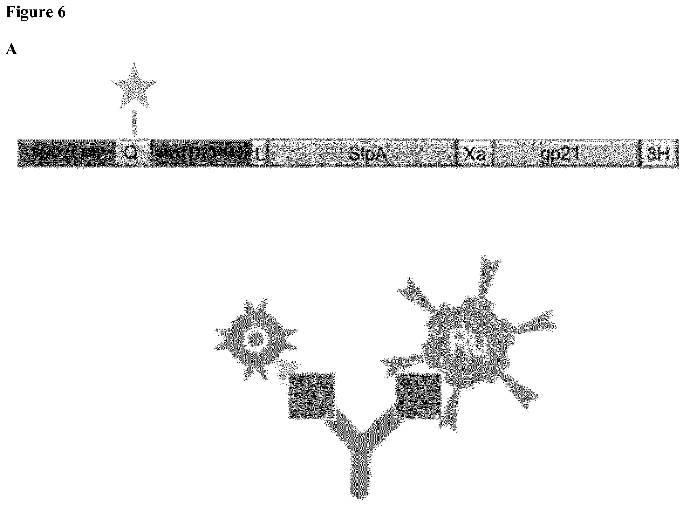

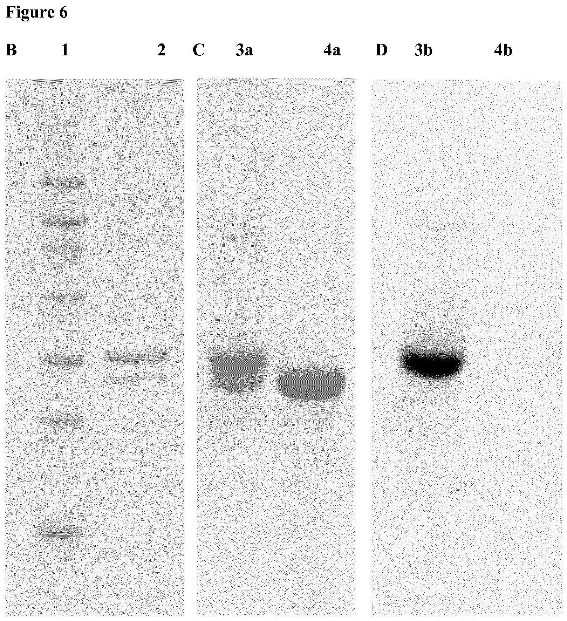

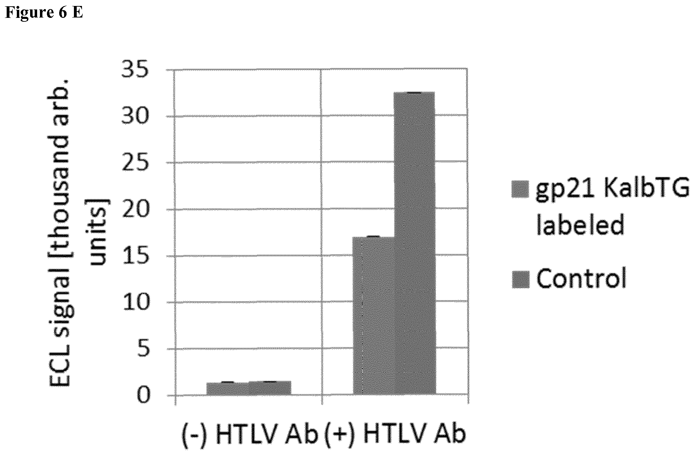

For results, see Figures 5 and 6.

**Mass spectrometric analysis of TtSlyQD-Xa-gp21-8H Ruthenium labeling**

Figure 5A schematically depicts the fusion protein 'TtSlyQD-Xa-gp21-8H' of SEQ ID NO:60. Figure 5B shows results after SDS-PAGE analysis showing specific and unspecific Ruthenium labeling of the recombinant gp21 (HTLV) antigen with the bacterial transglutaminase of *Streptomyces mobaraensis.* Depicted are unlabeled, single-labeled, double-labeled, and triple-labeled 'TtSlyQD-Xa-gp21-8H' fusion proteins after Ru labeling. Ru-label see in Figure 5C.

Two pooled fractions of the Ruthenium labelled fusion protein were analyzed, [1] TtSlyQD-Xa-gp21-8H_Fraction 17-19 and [2] TtSlyQDXa-gp21-8H_Fraction 20-23; unlabeled 'TtSlyQD-Xa-gp21-8H' protein served a control (TtSlyQD-Xa-gp21-8H_Control).

Peptide mapping was performed. After peptide mapping by digestion with Trypsin, chromatographic separation, subsequent MS/MS analysis, and database searching in combination with manual interpretation tools, the following was found:

(a) The tryptic peptide ,62-85' **"AYGAGSGGGGDYALQGGGGGSSGK"** SEQ ID NO:63 (numbering is referred to the sequence given below) with one label was found in both fractions 'TtSlyQD-Xa-gp21-8H_Fraction 17-19' and ,TtSlyQD-Xa-gp21-8H_Fraction 20-23'.

(b) The tryptic peptide '195-222' **"ALQEQAAFLNITNSHVSILQERPPLENR"** SEQ ID NO:64 with one label was found in traces only in ,TtSlyQD-Xa-gp21-8H_Fraction 17-19'.

(c) The tryptic peptide ,241-255' **"EALQTGGHHHHHHHH"** SEQ ID NO:65 (His-Tag) with one label was found in both fractions ,TtSlyQD-Xa-gp21-8H_Fraction 17-19' and ,TtSlyQD-Xa-gp21-8H_Fraction 20-23'.

(d) In addition to the above, also other Ruthenium labelled peptides/ byproducts were found to be present in both Fractions, however in insignificant quantities.

Amino acid sequence of TtSlyQD-Xa-gp21-8H, tryptic peptides as given above in (a-c) are marked in boldface and underlined:

MKVGQDKVVTIRYTLQVEGEVLDQGELSYLHGHRNLIPGLEEALEGREEGEAFQAH

VPAEK**AYGAGSGGGGDYALQGGGGGSSGK**DLDFQVEVVKVREATPEELLHGHAH

GGGSGGGSGGGSGGGSGGGSGGGIEGRMSLASGKSLLHEVDKDISQLTQAIVKNHKN

LLKIAQYAAQNRRGLDLLFWEQGGLAK**ALQEQAAFLNITNSHVSILQERPPLENR**V

LTGWGLNWDLGLSQWAR**EALQTGGHHHHHHHH** (SEQ ID NO:60)

**Recombinant gp21 (HTLV) antigen, site-specifically labeled with Biotin and Ruthenium using the bacterial transglutaminase of Kutzneria albida demonstrates the advantages of the invention in the Elecsys HTLV-I/II in-vitro diagnostic assay.**

The results are presented in Figure 6 and the following.

Figure 6 A depicts schematic representations showing the primary structure of the Ruthenium and Biotin labeled gp21 antigen (Q = KalbTG Q-tag, L = linker, Xa = factor Xa cleavage site, 8H = His-tag) and the diagnostic test principle; Serum Antibody from a patient sample (red) is bridging Biotin and Ruthenium labeled antigens (green).

Figure 6 B-D: SDS-PAGE analysis showing single Biotin or Ruthenium labeling of gp21 with KalbTG. B: Coomassie stained SDS-PAGE gel showing Biotin Labeling, lane 1: prestained protein molecular weight marker, lane 2: TtSlyKQD-SlpA-Xa-gp21-8H incubated with KalbTG and biotin label. C: Coomassie stained SDS-PAGE gel showing Ruthenium labeling, lane 3a: TtSlyKQD-SlpA-Xa-gp21-8H incubated with KalbTG and Ruthenium label, lane 4a: Unmodified TtSlyKQD-SlpA-Xa-gp21-8H (control). D: Fluorescence image of the SDS-PAGE analysis shown in C. Lanes 3b-4b correspond to lanes 3a-4a.

Figure 6 E: Elecsys assay (HTLV-I/II) on sera negative (left) and positive (right) for HTLV antibody using the MTG labeled reagents or the commerical kit (control) reagents. Note the positive control signal is higher than KalbTG labeled gp21 signal, since the labeling stoichiometry in the control is higher than 1:1. That is to say, the KalbTG labeled molecule carries less label. Measurements were performed in duplicates, shown is the average signal.

The a mino acid sequence of TtSlyKQD-SlpA-Xa-gp21-8H is as follows:

MKVGQDKVVTIRYTLQVEGEVLDQGELSYLHGHRNLIPGLEEALEGREEGEAFQAH

VPAEKAYGAGSGGGGYRYRQGGGGGSSGKDLDFQVEVVKVREATPEELLHGHAHG

GGSGGGSGGGSGGGSGGGSGGGMSESVQSNSAVLVHFTLKLDDGTTAESTRNNGKP

ALFRLGDASLSEGLEQHLLGLKVGDKTTFSLEPDAAFGVPSPDLIQYFSRREFMDAGE

PEIGAIMLFTAMDGSEMPGVIREINGDSITVDFNHPLAGQTVHFDIEVLEIDPALEAGG

GSGGGSGGGSGGGSGGGIEGRMSLASGKSLLHEVDKDISQLTQAIVKNHKNLL

KIAQYAAQNRRGLDLLFWEQGGLAKALQEQAAFLNITNSHVSILQERPPLENRVLTG

WGLNWDLGLSQWAREALQTGGHHHHHHHH (SEQ ID NO:62).

**Claims**

1. Recombinant transglutaminase (TG) substrate according to the following general formula I

$$(F^*\text{-}L)_y\text{-}X \qquad (I)$$

wherein

F* is selected from an amino acid sequence of the FKBP domain which comprises the N-terminal amino acids 1 to 64 and 123 to 149 of the SLYD polypeptide , wherein amino acids 65 to 122 are replaced by an amino acid sequence ("Q-tag") of 5 to 15 amino acids, the Q-tag comprising a sub-sequence of 5 contiguous amino acids having at least 80% sequence identity to the YRYRQ portion of the peptide sequence $X_1$-YRYRQ-$X_2$ (SEQ ID NO. 1), wherein $X_1$ and $X_2$ are absent or constitute linker amino acids, wherein the Q-tag is an amino acid sequence selected from the group consisting of SEQ ID NO:8, SEQ ID NO:11, SEQ ID NO:18 and SEQ ID NO:20;
L is absent or is selected from a linker amino acid sequence; and
X is a protein of interest selected from an enzyme, an antigen, such as a viral protein, an antibody or fragment thereof, and other immunological binding partners;
y is an integer of between 1 and 100,
and wherein said TG substrate is a substrate for the TG function of the *Kutzneria albida* TG according to SEQ ID No. 23.

2. The recombinant transglutaminase (TG) substrate according to claim 1, wherein said linker sequence L comprises between 1 to 20 amino acids, wherein preferably said amino acids do not interfere essentially with the FKBP domain and/or the protein of interest.

3. An in vitro method for labelling a protein of interest selected from an enzyme, an antigen, such as a viral protein, an antibody or fragment thereof, and other immunological binding partners, comprising

a) providing the recombinant transglutaminase (TG) substrate according to any one of claims 1 or 2,
b) providing an effective amount of the transglutaminase of *Kutzneria albida,* preferably according to SEQ ID No. 23,
c) providing a suitable label linked comprising an alkyl-amine group, such as, for example, a lysine, and
d) contacting said components according to a) to c), whereby said transglutaminase attaches said label to said substrate.

4. The method according to claim 3, wherein said transglutaminase of *Kutzneria albida* is recombinantly produced.

5. The method according to claim 3 or 4, wherein said label is selected from an enzyme, biotin, a radioactive group, a dye, such as a fluorescent dye, an isotope, a chemiluminescent label, and a metal.

6. The method according to any one of claims 3 to 5, wherein said labeling is achieved in a stoichiometric ratio of label and protein of interest, for example at about 1:1.

7. A pharmaceutical or diagnostic composition comprising at least one labeled protein of interest as produced according to a method according to any one of claims 3 to 6, together with pharmaceutically acceptable carrier compounds.

8. The pharmaceutical or diagnostic composition according to claim 7, wherein said protein of interest is labelled at a stoichiometric ratio of label and protein of interest of, for example, about 1:1.

9. A diagnostic kit, comprising the diagnostic composition according to claim 7 or 8, optionally together with other components for performing an immunoassay.


**Patentansprüche**

1. Rekombinantes Transglutaminase(TG)-Substrat gemäß der folgenden allgemeinen Formel I:

$$(F^*\text{-}L)_y\text{-}X \qquad (I)$$

wobei F* aus einer Aminosäuresequenz der FKBP-Domäne ausgewählt ist, die die N-terminalen Aminosäuren 1 bis 64 und 123 bis 149 des SLYD-Polypeptids umfasst, wobei die Aminosäuren 65 bis 122 durch eine Aminosäuresequenz ("Q-Tag") von 5 bis 15 Aminosäuren ersetzt sind, wobei das Q-Tag eine Teilsequenz von 5 zusammenhängenden Aminosäuren umfasst, die wenigstens 80 % Sequenzidentität mit dem YRYRQ-Anteil der Peptidsequenz $X_1$-YRYRQ-$X_2$ (SEQ ID NO. 1) aufweisen, wobei $X_1$ und $X_2$ nicht vorhanden sind oder Verknüpferaminosäuren darstellen, wobei das Q-Tag eine Aminosäuresequenz ist, die aus der Gruppe ausgewählt ist, die aus SEQ ID NO: 8, SEQ ID NO: 11, SEQ ID NO: 18 und SEQ ID NO: 20 besteht;

L nicht vorhanden ist oder aus einer Verknüpferaminosäure ausgewählt ist; und
X ein Protein von Interesse ist, das aus einem Enzym, einem Antigen, wie etwa einem viralen Protein, einem Antikörper oder einem Fragment davon, und anderen immunologischen Bindungspartnern ausgewählt ist;
y eine ganze Zahl zwischen 1 und 100 ist, und wobei das TG-Substrat ein Substrat für die TG-Funktion der *Kutzneria-albida-TG* gemäß SEQ ID No. 23 ist.

2. Rekombinantes Transglutaminase(TG)-Substrat nach Anspruch 1, wobei die Verknüpfersequenz L zwischen 1 und 20 Aminosäuren umfasst, wobei vorzugsweise die Aminosäuren nicht wesentlich mit der FKBP-Domäne und/oder dem Protein von Interesse interferieren.

3. *In-vitro*-Verfahren zum Markieren eines Proteins von Interesse, das aus einem Enzym, einem Antigen, wie etwa einem viralen Protein, einem Antikörper oder einem Fragment davon, und anderen immunologischen Bindungspartnern ausgewählt ist, das Folgendes umfasst:

a) Bereitstellen des rekombinanten Transglutaminase(TG)-Substrats nach einem der Ansprüche 1 oder 2,
b) Bereitstellen einer wirksamen Menge der Transglutaminase von *Kutzneria albida*, vorzugsweise gemäß SEQ ID No. 23,
c) Bereitstellen einer geeigneten Markierung, die eine Alkylamingruppe, wie etwa zum Beispiel ein Lysin, umfasst, und
d) Inberührungbringen der Komponenten gemäß a) bis c), wodurch die Transglutaminase die Markierung an das Substrat anbringt.

4. Verfahren nach Anspruch 3, wobei die Transglutaminase von *Kutzneria albida* rekombinant hergestellt wird.

5. Verfahren nach Anspruch 3 oder 4, wobei die Markierung aus einem Enzym, Biotin, einer radioaktiven Gruppe, einem Farbstoff, wie etwa einem Fluoreszenzfarbstoff, einem Isotop, einer chemilumineszenten Markierung und einem Metall ausgewählt ist.

6. Verfahren nach einem der Ansprüche 3 bis 5, wobei das Markieren in einem stöchiometrischen Verhältnis von Markierung und Protein von Interesse, zum Beispiel, bei etwa 1 : 1 erreicht wird.

7. Pharmazeutische oder diagnostische Zusammensetzung, die wenigstens ein markiertes Protein von Interesse, das gemäß einem Verfahren nach einem der Ansprüche 3 bis 6 hergestellt wird, zusammen mit pharmazeutisch unbedenklichen Trägerverbindungen umfasst.

8. Pharmazeutische oder diagnostische Zusammensetzung nach Anspruch 7, wobei das Protein von Interesse in einem stöchiometrischen Verhältnis von Markierung und Protein von Interesse von, zum Beispiel, etwa 1 : 1 markiert ist.

9. Diagnostischer Kit, der die diagnostische Zusammensetzung nach Anspruch 7 oder 8 umfasst, optional zusammen mit anderen Komponenten zum Durchführen eines Immunoassays.

**Revendications**

1. Substrat de transglutaminase recombinante (TG) selon la formule générale I suivante

$$(F^*\text{-L})_y\text{-X} \qquad (I)$$

F* étant choisi parmi une séquence d'acides aminés du domaine FKBP qui comprend les acides aminés à extrémité N-terminale 1 à 64 et 123 à 149 du polypeptide SLYD, les acides aminés 65 à 122 étant remplacés par une séquence d'acides aminés (« étiquette Q ») de 5 à 15 acides aminés, l'étiquette Q comprenant une sous-séquence de 5 acides aminés contigus ayant au moins 80 % d'identité de séquence avec la partie YRYRQ de la séquence peptidique $X_1$-YRYRQ-$X_2$(SEQ ID N° 1), $X_1$ et $X_2$ étant absents ou constituant des acides aminés lieurs, l'étiquette Q étant une séquence d'acides aminés choisie dans le groupe constitué par SEQ ID N° : 8, SEQ ID N° : 11, SEQ ID N °: 18 et SEQ ID N° : 20 ;
L étant absent ou étant choisi parmi une séquence d'acides aminés lieurs ; et
X étant une protéine d'intérêt choisie parmi une enzyme, un antigène, tel qu'une protéine virale, un anticorps ou un fragment de celui-ci, et d'autres partenaires de liaison immunologique ;
y étant un nombre entier compris entre 1 et 100, et ledit substrat de TG étant un substrat pour la fonction TG de TG de *Kutzneria albida* selon SEQ ID N° 23.

2. Substrat de transglutaminase recombinante (TG) selon la revendication 1, ladite séquence de liaison L comprenant entre 1 et 20 acides aminés, de préférence lesdits acides aminés n'interférant pas essentiellement avec le domaine FKBP et/ou la protéine d'intérêt.

3. Procédé in vitro permettant de marquer une protéine d'intérêt choisie parmi une enzyme, un antigène, tel qu'une protéine virale, un anticorps ou un fragment de celui-ci, et d'autres partenaires de liaison immunologique, comprenant

   a) la fourniture du substrat de transglutaminase recombinante (TG) selon l'une quelconque des revendications 1 ou 2,
   b) la fourniture d'une quantité efficace de la transglutaminase de *Kutzneria albida,* de préférence selon SEQ ID N° 23,
   c) la fourniture d'un marqueur approprié lié comprenant un groupe alkyl-amine, tel que, par exemple, une lysine, et
   d) la mise en contact desdits composants selon a) à c), ladite transglutaminase fixant ledit marqueur audit substrat.

4. Procédé selon la revendication 3, ladite transglutaminase de *Kutzneria albida* étant produite par recombinaison.

5. Procédé selon la revendication 3 ou 4, ledit marqueur étant choisi parmi une enzyme, la biotine, un groupe radioactif, un colorant, tel qu'un colorant fluorescent, un isotope, un marqueur chimioluminescent et un métal.

6. Procédé selon l'une quelconque des revendications 3 à 5, ledit marquage étant obtenu dans un rapport stoechiométrique du marqueur et de la protéine d'intérêt, par exemple d'environ 1:1.

7. Composition pharmaceutique ou diagnostique comprenant au moins une protéine d'intérêt marquée telle que produite selon un procédé selon l'une quelconque des revendications 3 à 6, conjointement avec des composés de support pharmaceutiquement acceptable.

8. Composition pharmaceutique ou diagnostique selon la revendication 7, ladite protéine d'intérêt étant marquée selon un rapport stœchiométrique du marqueur et de la protéine d'intérêt, par exemple d'environ 1:1.

9. Trousse de diagnostic, comprenant la composition de diagnostic selon la revendication 7 ou 8, éventuellement conjointement avec d'autres composants pour effectuer un dosage immunologique.

**Figure 1**

**A) SEQ ID NO: 23**

```
  1 mhkwflraav vaavgfglpt liattaqaaa vaaptprapl applaedrsy rtwrvedyve
 61 aweryhgrem tederenlar gcigvtvvnl nredlsnppl nlsfgslrta eavqaalnki
121 vdthpspaqy eaavakdpil krlknvvkal pswidsaklk asifskrfys wqnpdwseer
181 ahttyrpdre tdqvdmstyr yrarpgyvnf dygwfdqdtn twwhanheep rmvvyqstlr
241 hysrplqdfd eqvftvafak kd
```

**B) SEQ ID NO: 24 (E. coli)**

```
            10         20         30         40         50
MKVAKDLVVS LAYQVRTEDG VLVDESPVSA PLDYLHGHGS LISGLETALE
            60         70         80         90        100
GHEVGDKFDV AVGANDAYGQ YDENLVQRVP KDVFMGVDEL QVGMRFLAET
           110        120        130        140        150
DQGPVPVEIT AVEDDHVVVD GNHMLAGQNL KFNVEVVAIR EATEEELAHG
           160        170        180        190
HVHGAHDHHH DHDHDGCCGG HGHDHGHEHG GEGCCGGKGN GGCGCH
```

**C) SEQ ID NO: 25 (Cyclobacteriaceae bacterium AK24)**

```
            10         20         30         40         50
MRFQSQLVFM EISENTVVGL TYELKVTNNE EDSIPFSVEV RDEEDPFYFV
            60         70         80         90        100
FGNSGLPEKF ERLLENKVAG NTFNFTLSIE EAYGHADEEL ILTVPKKQFT
           110        120        130        140        150
GEKGFEPEML EEGNFLPLID EDGYPMQAKV IKDLGEELLL DFNHPLVGMN
           160        170
LHFDGEVYKV RKATKEETEK GYIEVN
```

**D) SEQ ID NO: 26 (Bacteroidales bacterium CF)**

```
            10         20         30         40         50
MKIGTNKVVS LAYTLEVEGD VMETVTSEKP LEFIFGTGYL LPKFEENVSN
            60         70         80         90        100
KVVGDAFDFT LTASEGYGEE NPDAIIELPK DIFKVDGKIE EGLLTVGNIL
           110        120        130        140        150
PMQDSDGNRL QGSIDEIKDD VVVMNFNHPL AGADLHFKGI VVAVREASET
           160        170
ELVNGLRGEL GTSCGDGGCS GCSGCH
```

**Figure 2**

A

B

Figure 3

## TtSlyD-Qtag-gp21 Ruthenium labeling with KalbTG to specific Q-tag (YRYRQ):

A        Coomassie  Fluorescence

2 x labeled ⟶
1 x labeled ⟶
unlabeled ⟶

B

| SlyD (1-64) | Q | SlyD (123-149) | Xa | gp21 | 8H |

(labeling site confirmed by neg. control

Labeling with EcSlyD2-KalbTG and KalbTG_Ktag_Ru(1-6)[1-Z: 6-PEG27-Lys(BPRu)] (DXRR, BMO 28.430948)
45 min, 37 °C

Figure 4

**Figure 5**

A

B

**Figure 5 C**

# Ruthenium-Label

Sequence

\*Z-
\*RSKLG\*O2Oc\*EUEUEUEUEUEUEUEUEUEUEUEUEUEUEUEUEUEUEUE
UEUK\*BPRu-\*\*-OH\*

Structure

**Figure 6**

**A**

Figure 6

Figure 6 E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014071978 A
- WO 0043492 A
- WO 2012059882 A
- US 20160178627 A
- WO 2016096785 A
- EP 15200111

### Non-patent literature cited in the description

- *Methods Mol Biol.,* 2011, vol. 705, 211-24
- **STANDAERT, R.F. et al.** *Nature,* 1990, vol. 346, 671-674
- **IDENO, A. et al.** *Appl. Microbiol. Biotechnol.,* 2004, vol. 64, 99-105
- **SCHOLZ, C et al.** *J. Biol. Chem.,* 1996, vol. 271, 12703-12707
- **LOW C et al.** *J. Mol. Biol.,* 2010, vol. 398, 375-390
- **KOVERMANN M ; SCHMID FX ; BALBACH J.** Molecular function of the prolyl cis/trans isomerase and metallochaperone SlyD. *Biol Chem.,* August 2013, vol. 394 (8), 965-75
- **DECENZO, M.T. et al.** *Protein Eng.,* 1996, vol. 9, 173-180
- **BRECHT, S. et al.** *Neuroscience,* 2003, vol. 120, 1037-1048
- **SCHORIES, B. et al.** *J. Pept. Sci.,* 2007, vol. 13, 475-480
- **TIMERMAN, A.P. et al.** *J. Biol. Chem.,* 1995, vol. 270, 2451-2459
- **GEITNER AJ ; VARGA E ; WEHMER M ; SCHMID FX.** Generation of a highly active folding enzyme by combining a parvulin-type prolyl isomerase from SurA with an unrelated chaperone domain. *J Mol Biol.,* 15 November 2013, vol. 425 (22), 4089-98
- **KNAPPE TA ; ECKERT B ; SCHAARSCHMIDT P ; SCHOLZ C ; SCHMID FX.** Insertion of a chaperone domain converts FKBP12 into a powerful catalyst of protein folding. *J Mol Biol.,* 18 May 2007, vol. 368 (5), 1458-68
- **CHICHILI et al.** *Linkers in the structural biology of protein-protein interactions Protein Sci.,* February 2013, vol. 22 (2), 153-167
- database. WP_030111976